(19) Europäisches Patentamt
European Patent Office
Office européen des brevets

(11) EP 3 636 284 A1

(12) EUROPEAN PATENT APPLICATION

(43) Date of publication:
15.04.2020 Bulletin 2020/16

(51) Int Cl.:
*A61K 47/68* (2017.01) *A61P 35/00* (2006.01)

(21) Application number: 18199879.0

(22) Date of filing: 11.10.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: NBE Therapeutics AG
4057 Basel (CH)

(72) Inventors:
- GRAWUNDER, Ulf
  4423 Hersberg (CH)
- BEERLI, Roger
  8106 Adlikon bei Regensdorf (CH)
- WALDMEIER, Lorenz
  4058 Basel (CH)
- PRETTO, Francesca
  8051 Zürich (CH)

(74) Representative: Michalski Hüttermann & Partner
Patentanwälte mbB
Speditionstraße 21
40221 Düsseldorf (DE)

(54) **BINDING PROTEIN-TOXIN CONJUGATES COMPRISING ANTHRACYCLINES, AND USE THEREOF IN IMMUNE-ONCOLOGICAL APPLICATIONS**

(57) The present invention relates to binding protein-toxin conjugates comprising one or more anthracycline toxin moieties, and the use thereof in immunooncological applications.

The present invention also claims their use in combination with an immune checkpoint inhibitor.

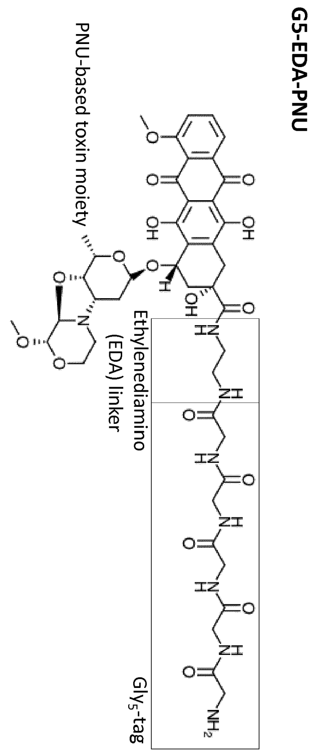


Fig. 1

EP 3 636 284 A1

SEQUENCE LISTING

<110> NBE Therapeutics

<120> Binding protein-toxin conjugates comprising anthracyclines, and use thereof in immune-oncological applications

<130> ND40705

<160> 21

<170> PatentIn version 3.5

<210> 1
<211> 447
<212> PRT
<213> XBR1-402 HC amino acid sequence

<400> 1

```
Gln Glu Gln Gln Lys Glu Ser Gly Gly Gly Leu Phe Lys Pro Thr Asp
1               5                   10                  15

Thr Leu Thr Leu Thr Cys Thr Ala Ser Gly Phe Asp Ile Ser Ser Tyr
            20                  25                  30

Tyr Met Ser Trp Val Arg Gln Ala Pro Gly Asn Gly Leu Glu Trp Ile
        35                  40                  45

Gly Ala Ile Gly Ile Ser Gly Asn Ala Tyr Tyr Ala Ser Trp Ala Lys
    50                  55                  60

Ser Arg Ser Thr Ile Thr Arg Asn Thr Asn Leu Asn Thr Val Thr Leu
65                  70                  75                  80

Lys Met Thr Ser Leu Thr Ala Ala Asp Thr Ala Thr Tyr Phe Cys Ala
                85                  90                  95

Arg Asp His Pro Thr Tyr Gly Met Asp Leu Trp Gly Pro Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175
```

```
Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430
```

```
His Asn His Tyr Thr Gln Lys Ser Leu Ser Leu Ser Pro Gly Lys
        435                 440                 445


<210>  2
<211>  212
<212>  PRT
<213>  XBR1-402 LC amino acid sequence

<400>  2

Ser Tyr Glu Leu Thr Gln Leu Pro Ser Val Ser Val Ser Leu Gly Gln
1               5                   10                  15

Thr Ala Arg Ile Thr Cys Glu Gly Asn Asn Ile Gly Ser Lys Ala Val
            20                  25                  30

His Trp Tyr Gln Gln Lys Pro Gly Leu Ala Pro Gly Leu Leu Ile Tyr
        35                  40                  45

Asp Asp Asp Glu Arg Pro Ser Gly Val Pro Asp Arg Phe Ser Gly Ser
    50                  55                  60

Asn Ser Gly Asp Thr Ala Thr Leu Thr Ile Ser Gly Ala Gln Ala Gly
65                  70                  75                  80

Asp Glu Ala Asp Tyr Tyr Cys Gln Val Trp Asp Ser Ser Ala Tyr Val
                85                  90                  95

Phe Gly Gly Gly Thr Gln Leu Thr Val Thr Gly Gln Pro Lys Ala Ala
            100                 105                 110

Pro Ser Val Thr Leu Phe Pro Pro Ser Ser Glu Glu Leu Gln Ala Asn
        115                 120                 125

Lys Ala Thr Leu Val Cys Leu Ile Ser Asp Phe Tyr Pro Gly Ala Val
    130                 135                 140

Thr Val Ala Trp Lys Ala Asp Ser Ser Pro Val Lys Ala Gly Val Glu
145                 150                 155                 160

Thr Thr Thr Pro Ser Lys Gln Ser Asn Asn Lys Tyr Ala Ala Ser Ser
                165                 170                 175

Tyr Leu Ser Leu Thr Pro Glu Gln Trp Lys Ser His Lys Ser Tyr Ser
            180                 185                 190

Cys Gln Val Thr His Glu Gly Ser Thr Val Glu Lys Thr Val Ala Pro
        195                 200                 205
```

```
Thr Glu Cys Ser
    210


<210>  3
<211>  447
<212>  PRT
<213>  Ac10 HC amino acid sequence

<400>  3

Gln Ile Gln Leu Gln Gln Ser Gly Pro Glu Val Val Lys Pro Gly Ala
1               5                   10                  15

Ser Val Lys Ile Ser Cys Lys Ala Ser Gly Tyr Thr Phe Thr Asp Tyr
            20                  25                  30

Tyr Ile Thr Trp Val Lys Gln Lys Pro Gly Gln Gly Leu Glu Trp Ile
        35                  40                  45

Gly Trp Ile Tyr Pro Gly Ser Gly Asn Thr Lys Tyr Asn Glu Lys Phe
    50                  55                  60

Lys Gly Lys Ala Thr Leu Thr Val Asp Thr Ser Ser Ser Thr Ala Phe
65                  70                  75                  80

Met Gln Leu Ser Ser Leu Thr Ser Glu Asp Thr Ala Val Tyr Phe Cys
                85                  90                  95

Ala Asn Tyr Gly Asn Tyr Trp Phe Ala Tyr Trp Gly Gln Gly Thr Gln
            100                 105                 110

Val Thr Val Ser Ala Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205
```

Thr Lys Val Asp Lys Lys Val Glu Pro Lys Ser Cys Asp Lys Thr His
210 215 220

Thr Cys Pro Pro Cys Pro Ala Pro Glu Leu Leu Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Pro Ala Pro Ile Glu Lys Thr Ile
325 330 335

Ser Lys Ala Lys Gly Gln Pro Arg Glu Pro Gln Val Tyr Thr Leu Pro
340 345 350

Pro Ser Arg Asp Glu Leu Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 4

<211> 218
<212> PRT
<213> Ac10 LC amino acid sequence

<400> 4

```
Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Phe Asp
            20                  25                  30

Gly Asp Ser Tyr Met Asn Trp Tyr Gln Gln Lys Pro Gly Gln Pro Pro
        35                  40                  45

Lys Val Leu Ile Tyr Ala Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Trp Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 5

<211> 448
<212> PRT
<213> 2A2 HC amino acid sequence

<400> 5

```
Gln Val Gln Leu Gln Gln Ser Gly Ala Glu Leu Val Arg Pro Gly Ala
1               5                   10                  15

Ser Val Thr Leu Ser Cys Lys Ala Ser Gly Tyr Thr Phe Ser Asp Tyr
            20                  25                  30

Glu Met His Trp Val Ile Gln Thr Pro Val His Gly Leu Glu Trp Ile
        35                  40                  45

Gly Ala Ile Asp Pro Glu Thr Gly Gly Thr Ala Tyr Asn Gln Lys Phe
    50                  55                  60

Lys Gly Lys Ala Ile Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Gly Tyr Tyr Asp Tyr Asp Ser Phe Thr Tyr Trp Gly Gln Gly Thr
            100                 105                 110

Leu Val Thr Val Ser Ala Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
```

```
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  6
<211>  214
<212>  PRT
<213>  2A2 LC amino acid sequence

<400>  6

Asp Ile Val Met Thr Gln Ser Gln Lys Ile Met Ser Thr Thr Val Gly
```

1 5 10 15

Asp Arg Val Ser Ile Thr Cys Lys Ala Ser Gln Asn Val Asp Ala Ala
20 25 30

Val Ala Trp Tyr Gln Gln Lys Pro Gly Gln Ser Pro Lys Leu Leu Ile
35 40 45

Tyr Ser Ala Ser Asn Arg Tyr Thr Gly Val Pro Asp Arg Phe Thr Gly
50 55 60

Ser Gly Ser Gly Thr Asp Phe Thr Leu Thr Ile Ser Asn Met Gln Ser
65 70 75 80

Glu Asp Leu Ala Asp Tyr Phe Cys Gln Gln Tyr Asp Ile Tyr Pro Tyr
85 90 95

Thr Phe Gly Gly Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 7
<211> 5
<212> PRT
<213> Staphylococcus aureus sortase A recognition sequence 1, with X being any amino acid

<220>

<221> Xaa
<222> (3)..(3)
<223> Xaa can be any amino acid

<400> 7

Leu Pro Xaa Thr Gly
1 5

<210> 8
<211> 5
<212> PRT
<213> Staphylococcus aureus sortase A recognition sequence 2, with X being any amino acid

<220>
<221> Xaa
<222> (3)..(3)
<223> Xaa can be any amino acid

<400> 8

Leu Pro Xaa Ala Gly
1 5

<210> 9
<211> 5
<212> PRT
<213> recognition sequence for Staphylococcus aureus sortase A or engineered sortase A 4S-9 from Staphylococcus aureu

<220>
<221> Xaa
<222> (3)..(3)
<223> Xaa can be any amino acid

<400> 9

Leu Pro Xaa Ser Gly
1 5

<210> 10
<211> 5
<212> PRT
<213> recognition sequence for engineered sortase A 2A-9 from Staphylococcus aureus, with X being any amino acid

<220>
<221> Xaa
<222> (3)..(3)
<223> Xaa can be any amino acid

<400> 10

Leu Ala Xaa Thr Gly
1 5

<210> 11
<211> 5
<212> PRT
<213> Streptococcus pyogenes sortase A recognition sequence, with X being any amino acid

<220>
<221> Xaa
<222> (3)..(3)
<223> Xaa can be any amino acid

<400> 11

Leu Pro Xaa Thr Ala
1 5

<210> 12
<211> 5
<212> PRT
<213> Staphylococcus aureus sortase recognition sequence

<400> 12

Asn Pro Gln Thr Asn
1 5

<210> 13
<211> 5
<212> PRT
<213> Linker derived from Staphylococcus aureus sortase A recognition sequence 1, with X being any amino acid and n ? 1 and ? 21

<220>
<221> Xaa
<222> (3)..(3)
<223> Xaa can be any amino acid

<400> 13

Leu Pro Xaa Thr Gly
1 5

<210> 14
<211> 5
<212> PRT
<213> Linker derived from Staphylococcus aureus sortase A recognition sequence 2, with X being any amino acid and n ? 1 and ? 21

<220>
<221> Xaa
<222> (3)..(3)
<223> Xaa can be any amino acid

<400> 14

Leu Pro Xaa Ala Gly

1 5

<210> 15
<211> 5
<212> PRT
<213> Linker derived from recognition sequence for Staphylococcus aureus sortase A or engineered sortase A 4S-9 from Staphylococcus aureus, with X being any amino acid and n ? 1 and ? 21

<220>
<221> Xaa
<222> (3)..(3)
<223> Xaa can be any amino acid

<400> 15

Leu Pro Xaa Ser Gly
1 5

<210> 16
<211> 5
<212> PRT
<213> Linker derived from recognition sequence for engineered sortase A 2A-9 from Staphylococcus aureus, with X being any amino acid and n ? 1 and ? 21

<220>
<221> Xaa
<222> (3)..(3)
<223> Xaa can be any amino acid

<400> 16

Leu Ala Xaa Thr Gly
1 5

<210> 17
<211> 5
<212> PRT
<213> Linker derived from Streptococcus pyogenes sortase A recognition sequence, with X being any amino acid and n ? 1 and ? 21

<220>
<221> Xaa
<222> (3)..(3)
<223> Xaa can be any amino acid

<220>
<221> Xaa
<222> (5)..(5)
<223> Xaa is G or A

<400> 17

Leu Pro Xaa Thr Xaa
1 5

<210> 18
<211> 5
<212> PRT
<213> Linker derived from Staphylococcus aureus sortase recognition sequence, with n ? 1 and ? 21

<400> 18

Asn Pro Gln Thr Gly
1               5

<210> 19
<211> 19
<212> PRT
<213> leader sequence

<400> 19

Met Asn Phe Gly Leu Arg Leu Ile Phe Leu Val Leu Thr Leu Lys Gly
1               5                   10                  15

Val Gln Cys

<210> 20
<211> 447
<212> PRT
<213> huXBR1-402-17 HC amino acid sequence

<400> 20

Gln Val Gln Leu Arg Glu Ser Gly Pro Gly Leu Val Lys Pro Ser Glu
1               5                   10                  15

Thr Leu Ser Leu Thr Cys Thr Val Ser Gly Phe Asp Ile Ser Ser Tyr
            20                  25                  30

Tyr Met Ser Trp Val Arg Gln Pro Pro Gly Lys Gly Leu Glu Trp Ile
        35                  40                  45

Gly Ala Ile Gly Ile Ser Gly Asn Ala Tyr Tyr Ala Ser Trp Ala Lys
    50                  55                  60

Ser Arg Val Thr Ile Ser Arg Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp His Pro Thr Tyr Gly Met Asp Leu Trp Gly Pro Gly Thr Leu
            100                 105                 110

```
Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365
```

```
Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445


<210>  21
<211>  212
<212>  PRT
<213>  huXBR1-402-17 LC amino acid sequence

<400>  21

Ser Tyr Glu Leu Thr Gln Pro Pro Ser Val Ser Val Ala Pro Gly Lys
1               5                   10                  15

Thr Ala Arg Ile Thr Cys Glu Gly Asn Asn Ile Gly Ser Lys Ala Val
            20                  25                  30

His Trp Tyr Gln Gln Lys Pro Gly Gln Ala Pro Val Leu Val Ile Tyr
        35                  40                  45

Asp Asp Asp Glu Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ala Tyr Val
                85                  90                  95

Phe Gly Gly Gly Thr Lys Leu Thr Val Leu Gly Gln Pro Lys Ala Ala
            100                 105                 110

Pro Ser Val Thr Leu Phe Pro Pro Ser Ser Glu Glu Leu Gln Ala Asn
        115                 120                 125

Lys Ala Thr Leu Val Cys Leu Ile Ser Asp Phe Tyr Pro Gly Ala Val
    130                 135                 140
```

```
Thr Val Ala Trp Lys Ala Asp Ser Ser Pro Val Lys Ala Gly Val Glu
145                 150                 155                 160

Thr Thr Thr Pro Ser Lys Gln Ser Asn Asn Lys Tyr Ala Ala Ser Ser
                165                 170                 175

Tyr Leu Ser Leu Thr Pro Glu Gln Trp Lys Ser His Arg Ser Tyr Ser
            180                 185                 190

Cys Gln Val Thr His Glu Gly Ser Thr Val Glu Lys Thr Val Ala Pro
        195                 200                 205

Thr Glu Cys Ser
    210
```

## Description

### Field of the invention

The present invention relates to binding protein-toxin conjugates comprising one or more anthracycline toxin moieties, and the use thereof in immune-oncological applications.

### Background

Binding protein-toxin conjugates, with antibody-drug-conjugates (ADC) as their most prominent representatives, have had a tremendous impact in cancer therapy, providing new therapeutic approaches for tumor types, which so far could not be treated.

On the other hand, the discovery of immune checkpoints, and the development of immune checkpoint inhibitors, has again brought into the focus the role of the immune system in cancer therapy, and ways to stimulate the immune system, or to overcome immune checkpoints that the respective tumors use for self protection against immune system attacks.

Although both approaches have been discussed very enthusiastically, and have demonstrated significant progress in the treatment of cancer, it also became clear that there are tumor types which cannot be treated with these new approaches.

It has for example been shown that ADCs with particular toxins are not active against specific cancer types, although they address, by their antibody, a cancer specific antigen. It has also been shown that tumor types exist where immune checkpoint inhibitors have no efficacy.

It is hence one object of the present invention to provide new approaches which make tumor types treatable which could so far not be addressed by existing therapies.

It is one further object of the present invention to provide combination therapies which increase the anti-tumor activity of the corresponding mono therapies.

These and further objects are met with methods and means according to the independent claims of the present invention. The dependent claims are related to specific embodiments.

### Summary of the Invention

The present invention provides binding protein-toxin conjugates comprising one or more anthracycline toxin moieties, and the use thereof in immune-oncological applications. The invention and general advantages of its features will be discussed in detail below.

### Description of the Figures

**Figure 1.** Chemical structures of pentaglycine-modified EDA-anthracycline PNU-159682 derivative (G5-EDA-PNU or G5-PNU) used for the generation of site-specifically conjugated ADCs used in the experiments in this invention. G3-PNU and G2-PNU refer to the same structures but wherein the pentaglycine is replaced by a triglycine or a diglycine, respectively.

**Figure 2.** FACS analysis for expression of human ROR1 stably expressed on mouse EMT-6 breast cancer cells. EMT-6-ROR1 clone 14 selected for *in vivo* studies has been analyzed by FACS staining for ROR1 expression with the fluorescently labeled anti-RORl antibody clone 2A2. The negative control shows a staining of the same cells with a fluorescently labeled isotype-matched control antibody.

**Figure 3.** Dose titration of novel anti-RORl PNU-ADC, based on antibody XBR1-402, with a single treatment at different dose levels (0.25, 0.5 and 1 mg/kg) in orthotopic syngeneic breast cancer model EMT-6-ROR1 (clone 14). (A) Tumor growth of the orthotopically transplanted breast tumors is monitored in the different treatment groups over time as indicated. (B) tumor growth curves in individual mice of the groups displayed in panel (A).

**Figure 4.** Tumor volume evolution in orthotopically transplanted syngeneic mice with EMT-6-ROR1 (clone 14) breast cancer cells, either (A) with injection of vehicle control (PBS), or with (B) single administration of 0.25mg/kg with an isotype-matched control PNU-ADC comprising anti-CD30 antibody brentuximab, clone Ac10, or (C) single administration of 0.25mg/kg with anti-RORl PNU-ADC comprising novel anti-RORl antibody XBR1-402, which according

to Fig. 3 has been determined to be a suboptimal dose for treatment of orthotopic EMT-6/ROR1 breast tumors in mice. Panels (D) and (E) show combination treatments with anti-CTLA4 immune-checkpoint inhibitor antibody 9D9 with 0.25 mg/kg of isotype-matched control ADC Ac10 and 0.25 mg/kg novel anti-RORl-PNU ADC, as indicated. (F) Median group tumor volume evolution (with application of Last-Observation-Carried-Forward (LOCF) methodology) for the following groups: vehicle control, Ac10-G5-PNU isotype control and XBR1-402-G5-PNU, (G) Median group tumor volume evolution (with application of Last-Observation-Carried-Forward (LOCF) methodology) for the following groups: vehicle control, Ac10-G5-PNU isotype control and anti-mouse CTLA-4 antibody 9D9, and XBR1-402-G5-PNU and anti-mouse CTLA-4 antibody 9D9.

**Figure 5.** FACS analysis for expression of human ROR1 stably expressed on (A) mouse CT-26 breast cancer cells (clone 3) and (B) B26 mouse leukemia cells (pool). CT-26-ROR1 clone 3 and B16-ROR1 pools selected for *in vivo* studies have been analyzed by FACS staining for ROR1 expression with the fluorescently labeled anti-RORl antibody clone XBR1-402 (right peak). The negative control shows a staining of the same cells with a fluorescently labeled isotype-matched control antibody (left peak).

**Figure 6.** Tumor volume evolution in mice transplanted with CT-26-ROR1 clone 3 tumor cells as per Example 6: (A) vehicle control (untreated) of group 1, (B) isotype control of group 2, (C) anti-RORl ADC huXBR1-402-17 of group 3, (D) isotype control plus immune checkpoint inhibitor anti-mouse PD-1 antibody of group 4, (E) anti-RORl ADC huXBR1-402-17 plus immune checkpoint inhibitor anti-mouse PD-1 antibody of group 7. The insets in panels A-E provide the number of mice in which tumor growth was prevented relative to number of mice in each treatment group. The results suggest a cooperation between the evaluated immune checkpoint inhibitor and the targeted PNU derivative-comprising ADC.

**Figure 7.** Lung surface colony volumes in mice following i.v. application of ROR1-overexpressing B16 pools, as per Example 7, following treatment with vehicle control ("vehicle", group 1), immune checkpoint inhibitor anti-mouse CTLA-4 antibody ("CTLA-4", group 2), anti-RORl ADC huXBR1-402-17 ("ADC", group 3), and anti-RORl ADC huXBR1-402-17 plus immune checkpoint inhibitor anti-mouse CTLA-4 antibody ("ADC + CTLA-4", group 4). These results suggest, in a poorly immunogenic tumor, a strong synergy between the evaluated immune checkpoint inhibitor and the targeted PNU derivative-comprising ADC. "n.s." means not statistically significantly different, "*" indicates statistically significantly different.

**Figure 8.** CD4 and CD8-staining of hRORl-overexpressing B16 tumor cryo-sections from (A) an untreated control mouse, and (B) the three mice treated with ADCs (once, at 4 mg/kg) as per the invention. As per Figure 8(A), B16 tumors naturally contain low levels of CD4+ and CD8+ cells, in keeping with the status of B16 tumors as cold tumors. Figure 8(B) shows that treatment with the ADC, as per the present invention, increases the number of CD4+ and CD8+ cells within the tumor.

**Detailed Description of the Invention**

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, to prior art documents that disclose standard or routine methods. In that case, the incorporation by reference has mainly the purpose to provide sufficient enabling disclosure, and avoid lengthy repetitions.

According to a first aspect of the invention, a binding protein-toxin conjugate is provided comprising one or more anthracycline toxin moieties conjugated to a binding protein

(for the manufacture of a medicament) for the treatment of a patient

- suffering from,
- being at risk of developing, and/or
- being diagnosed for

a neoplastic disease characterized as being a cold tumor.

According to a second aspect of the invention, a method of treating or preventing a neoplastic disease characterized as being a cold tumor is provided, said method comprising administering to a patient a binding protein-toxin conjugate comprising one or more anthracycline toxin moieties.

A "hot tumor" defines a cancerous or neoplastic tissue which is characterized by immune cell infiltration. The level of immune infiltration reflects whether the immune system is recognizing and engaging the tumor. The term "hot tumor" is synonymously used with the term "immunologically responsive tumor" herein. Alternatively, "hot tumors" may be defined as tumors that respond to treatment by immune checkpoint inhibitors.

As used herein, the term "respond to treatment" means a statistically significant reduction of tumor burden or growth relative to a comparable vehicle or isotype control treatment.

As used herein, the term "cold tumor" defines a cancerous or neoplastic tissue which is characterized by poor or lacking immune cell infiltration. The term "cold tumor" is synonymously used with the term "immunologically unresponsive tumor" herein.

One benchmark for a tumor to qualify as cold is the so-called Immunoscore, based on the density of two types of T cells in a sample of the tumor. One example is the density of two lymphocyte populations (CD3/CD45RO, CD3/CD8 or CD8/CD45RO), both in the core of the tumour (CT) and in the invasive margin (IM) of tumors. The Immunoscore provides a score ranging from Immunoscore 0 (I0) when low densities of both cell types are found in both regions, to Immunoscore 4 (I4), when high densities are found in both regions.

Methods to determine the Immunoscore have been described, e.g., in Galon et al. 2014, Pages et al 2009, Angell et al 2013, and Galon et al 2013, the contents of which are incorporated by reference herein.

So far, it has been discussed that tumors with a low Immunoscore ("cold tumors") are not very responsive to treatment with immune checkpoint inhibitors, like anti PD-1, anti CTLA-4 or anti-PD-Ll, while tumors with a high Immunoscore are responsive to such treatment (see Bu et al. 2016). Without being bound to theory, the rationale behind this finding could be that, if there is no immune engagement in a respective tumor, inhibition of an immune checkpoint by a given inhibitor cannot change anything to the better.

The inventors suggest that a binding protein-toxin conjugate comprising one or more anthracycline toxin moieties has particular immunologic efficacy in tumors, resulting, *inter alia,* in an immune protection from tumor re-challenge. This surprising finding could not be anticipated from the findings made with immune checkpoint inhibitors, like anti-PD-1, anti-CTLA-4 or anti-PD-Ll. The rationale that applies for the latter does not likely apply to binding protein-toxin conjugate comprising one or more anthracycline toxin moieties.

According to a third aspect of the invention, a combination of

(i) a binding protein-toxin conjugate comprising one or more anthracycline toxin moieties, conjugated to a binding protein, and
(ii) an immune checkpoint inhibitor

is provided (for the manufacture of a medicament) for the treatment of a patient

- suffering from,
- being at risk of developing, and/or
- being diagnosed for

a neoplastic disease characterized as being a cold tumor,
wherein the binding protein-toxin conjugate and the immune checkpoint inhibitor are administered to the patient simultaneously or sequentially, in any order.

In one embodiment, the binding protein-toxin conjugate comprising one or more anthracycline toxin is administered before the immune checkpoint inhibitor.

As used herein, the term "immune checkpoint inhibitor" refers to any binding agent or compound suitable to act against an immune-checkpoint protein.

An immune checkpoint is a protein that regulators the immune system, and are crucial for self-tolerance, which prevents the immune system from attacking cells indiscriminately, preferably selected from the group as disclosed in

the following table:

| Type | alias | Full name | Uni Prot |
|---|---|---|---|
| PD-1 | CD279 | Programmed cell death protein 1 | Q15116 |
| CTLA-4 | CD152 | Cytotoxic T-lymphocyte-associated Protein 4 | P16410 |
| PD-L1 | CD274, B7 Homolog 1 | Programmed cell death 1 ligand 1 | Q9NZQ7 |
| LAG3 | CD223 | Lymphocyte-activation gene 3 | P18627 |
| TIM3, | HAVCR2 | Hepatitis A virus cellular receptor 2 | Q8TDQ0 |
| OX40 | TNFRSF4, CD134 | Tumor necrosis factor receptor superfamily, member 4 | P43489 |
| OX40L | | ligand for CD134 | P23510 |
| CD112 | PVRL2, nectin-2 | Poliovirus receptor-related 2 | Q92692 |
| CD155 | | poliovirus receptor | P15151 |
| B7-H3 | CD276 | | Q5ZPR3 |
| B7-H4 | VTCN1 | V-set domain-containing T-cell activation inhibitor 1 | Q7Z7D3 |
| IDO1 | | Indolamin-2,3-Dioxygenase 1 | P14902 |
| IDO2 | | Indolamin-2,3-Dioxygenase 2 | Q6ZQW0 |
| TDO2 | | Tryptophan 2,3-dioxygenase | P48775 |
| TIGIT | WUCAM, Vstm3 | T cell immunoreceptor with Ig and ITIM domains | Q495A1 |
| Galectin-9 | | | O00182 |

Preferably, the interval within which the two different agents are administered to the patient is no longer than maximally four weeks.

According to a fourth aspect of the invention, a method of treating or preventing a neoplastic disease characterized as being a cold tumor is provided, said method comprising administering to a patient

(iii) a binding protein-toxin conjugate comprising one or more anthracycline toxin moieties, conjugated to a binding protein, and
(iv) an immune checkpoint inhibitor

wherein the binding protein-toxin conjugate and the immune checkpoint inhibitor are administered to the patient simultaneously or sequentially, in any order.

In one embodiment, the binding protein-toxin conjugate comprising one or more anthracycline toxins is administered before the immune checkpoint inhibitor.

As used herein, the term "cold tumor" defines a cancerous or neoplastic tissue which is characterized by a lack of, or insufficient, immune cell infiltration. The level of immune infiltration reflects whether the immune system is recognizing the tumor.

As discussed above, tumors with a low immunoscore ("cold tumors") are not very responsive to treatment with immune checkpoint inhibitors, like anti PD-1 or anti CTLA-4. The rationale behind this phenomenon could be that, if there is no immune system engagement in a respective tumor, inhibition of an immune checkpoint by a given inhibitor cannot change anything to the better.

In a preferred embodiment, a cold tumor is a tumor that does not respond to an immune checkpoint inhibitor when said immune checkpoint inhibitor is dosed *in vivo* as a monotherapy. Relative to an immunocompetent mouse model, a cold tumor is a tumor which, once established in the mouse, does not respond significantly (relative to a vehicle control group) to an immune checkpoint inhibitor dosed as a monotherapy at up to about 10 mg/kg, or up to 20 mg/kg, or up to the maximum tolerated dose (MTD) in that mouse model. In a human, a cold tumor is a tumor that does not respond significantly to an immune checkpoint inhibitor dosed as a monotherapy at up to 10 mg/kg, optionally with dosing every 2 to 3 weeks.

The inventors show that, with a treatment comprising (i) a binding protein-toxin conjugate comprising one or more anthracycline toxin moieties, conjugated to a binding protein, and (ii) an immune checkpoint inhibitor, such cold tumors can efficiently be treated.

This finding is surprising, and could not be anticipated from the findings made with immune checkpoint inhibitors, like anti PD-1, anti CTLA-4 or anti-PD-Ll therapies, nor with anthracycline-comprising binding protein-toxin conjugates. Without being bound to theory, it appears that the anthracycline-comprising binding protein-toxin conjugate is capable of rendering cold tumors susceptible to immune checkpoint therapy, i.e., transforming them into hot tumors, so that they can efficiently be treated with immune checkpoint inhibitors.

According to a first group of embodiments of the said aspects, the neoplastic disease is characterized as being a cold tumor characterized having an immunoscore of < 1.

According to another group of embodiments, the at least one anthracycline toxin moiety is a derivative of the anthracycline PNU-159682 having the following formula (i)

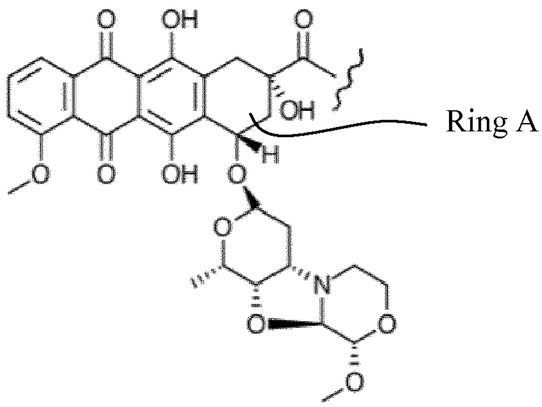

formula (i)

said toxin being conjugated at its wavy to the binding protein via a linker.

PNU-derived anthracyclines and the use thereof in antibody drug conjugates is enablingly disclosed in WO2016102679 assigned to the same applicant. The content of this publication is incorporated by reference herein.

According to another group of embodiments the Immune Checkpoint inhibitor is at least one selected from the group consisting of

- anti PD-1
- anti CTLA-4
- anti PD-L1
- anti LAG3
- anti TIM3, anti OX40/OX40L (CD134/CD134L),
- anti CD112
- anti CD155
- anti B7-H3
- anti B7-H4
- anti IDO1
- anti IDO2
- anti TDO2
- anti TIGIT, and/or

- anti Galectin-9.

Preferably, the anti PD-1 checkpoint inhibitor is a monoclonal antibody, more preferably nivolumab or pembrolizumab.

Preferably, the anti CTLA-4 checkpoint inhibitor is a monoclonal antibody, more preferably ipilimumab or tremelimumab.

Preferably, the anti PD-L1 checkpoint inhibitor is a monoclonal antibody, more preferably atezolizumab, durvalumab, avelumab or BMS-936559.

According to another group of embodiments, the conjugate comprises at its wavy line a linker structure X - $L_1$ - $L_2$ - $L_3$ - Y, wherein $L_1$ - $L_3$ represent linkers, and two of $L_1$ - $L_3$ are mandatory, and wherein X and Y further represent each one or more optional linkers.

According to another group of embodiments the linker structure comprises, as $L_2$, an oligo-glycine peptide $(Gly)_n$ coupled to said anthracycline derivative, directly or by means of another linker $L_1$, in such a way that the oligo-glycine $(Gly)_n$ peptide has a free amino terminus, and wherein n is an integer ≥ 1 and ≤ 21. It is important to understand that, in one specific embodiment (where Streptococcus pyogenes sortase A is used, see below), the oligo-glycine $(Gly)_n$ can optionally be replaced by an oligo-alanine $(Ala)_n$ or by mixed Gly/Ala peptides.

According to another group of embodiments, the oligo-glycine peptide $(Gly)_n$ is conjugated to the anthracycline derivative of formula (i) by means of an alkylenediamino linker (EDA), designated as $L_1$, which alkylenediamino linker is conjugated to the anthracycline derivative by means of a first amide bond, while it is conjugated to the carboxy terminus of the oligo-glycine peptide by means of a second amide bond, said conjugate of alkylenediamino linker and oligo-glycine peptide having the following formula (ii),

$$\wr\text{-NH-}(CH_2)_m\text{-NH-}(Gly)_n\text{-}NH_2 \qquad \text{formula (ii)}$$

wherein the wavy line indicates the linkage to the anthracycline derivative of formula (i), wherein m is an integer ≥ 1 and ≤ 11 and n is an integer ≥ 1 and ≤ 21. It is important to understand that, in one specific embodiment (where Streptococcus pyogenes sortase A is used, see below), the oligo-glycine $(Gly)_n$ can optionally be replaced by an oligo-alanine $(Ala)_n$.

According to another group of embodiments, the oligo-glycine peptide $(Gly)_n$ is, directly or by means of another linker $L_1$, coupled to Ring A of the anthracycline derivative of formula (ii) (where Ring A of the anthracycline core is as depicted in Shi et al., 2009). It is important to understand that, in one specific embodiment (where Streptococcus pyogenes sortase A is used, see below), the oligo-glycine $(Gly)_n$ can optionally be replaced by an oligo-alanine $(Ala)_n$.

According to another group of embodiments, the oligo-glycine peptide $(Gly_n)$ is conjugated to the anthracycline derivative of formula (i) by means of an alkyleneamino linker (EA), designated as $L_1$, which alkyleneamino linker is conjugated to the carboxy terminus of the oligo-glycine peptide by means of an amide bond, said conjugate of alkyleneamino linker and oligo-glycine peptide having the following formula (iii)

$$\wr\text{-}(CH_2)_m\text{-NH-}(Gly)_n\text{-}NH_2 \qquad \text{formula (iii)}$$

wherein the wavy line indicates the linkage to the anthracycline derivative of formula (i), wherein m is an integer ≥ 1 and ≤ 11 and n is an integer between ≥ 1 and ≤ 21. It is important to understand that, in one specific embodiment (where Streptococcus pyogenes sortase A is used, see below), the oligo-glycine $(Gly)_n$ can optionally be replaced by an oligo-alanine $(Ala)_n$.

According to another group of embodiments, the linker structure $L_3$ comprises a peptide motif that results from specific cleavage of a sortase enzyme recognition motif.

The sortase enzyme recognition motif serves as tag for the so-called sortase-enzyme mediated antibody conjugation (SMAC-technology), which is enablingly disclosed in WO2014140317 assigned to the same applicant. The content of this publication is incorporated by reference herein. In this technology, a sortase enzyme conjugates two proteins one of which is bearing such recognition motif, while the other one is bearing a oligo-glycine peptide $(Gly)_n$. It is important to understand that, in one specific embodiment (where Streptococcus pyogenes sortase A is used, see below), the oligo-glycine $(Gly)_n$ can optionally be replaced by an oligo-alanine $(Ala)_n$.

According to another group of embodiments, said sortase enzyme recognition motif comprises at least one of the following amino acid sequences: LPXTG, LPXAG, LPXSG, LAXTG, LPXTA or NPQTN, with X being any conceivable amino acid sequence.

According to another group of embodiments, the resulting linker has at least one of the following amino acid

sequences: -LPXTGn-, -LPXAGn-, -LPXSGn-, -LAXTGn-, -LPXTGn-, -LPXTAn- or -NPQTGn-, with Gn being an oligo-or polyglycine with n being an integer between ≥ 1 and ≤ 21, An being an oligo-or polyalanine with n being an integer between ≥ 1 and ≤ 21, and X being any conceivable amino acid sequence.

According to another group of embodiments, the anthracycline derivative is conjugated, by means of the one or more linkers, to the carboxy terminus of the binding protein, or to the carboxy terminus of at least one domain or subunit thereof.

According to another group of embodiments, the binding protein is conjugated to the free amino terminus of the oligo-glycine peptide ($Gly_n$) by means of an amide bond. It is important to understand that, in one specific embodiment (where Streptococcus pyogenes sortase A is used, see below), the oligo-glycine $(Gly)_n$ can optionally be replaced by an oligo-alanine $(Ala)_n$.

According to another group of embodiments, the binding protein is at least one selected from the group consisting of an antibody, an antibody-based binding protein, a modified antibody format retaining target binding capacity, an antibody derivative or a fragment retaining target binding capacity, an alternative scaffold and/or an antibody mimetic.

The terms "antibody", "antibody-based binding protein", "modified antibody format retaining target binding capacity", "antibody derivative or fragment retaining target binding capacity" refers to polypeptide chain(s) which exhibit a strong monovalent, bivalent or polyvalent binding to a given antigen, epitope or epitopes. Antibodies, antibody-based binding proteins and antigen-binding fragments used in the invention can be generated using any suitable technology, e.g., hybridoma technology, ribosome display, phage display, gene shuffling libraries, semi-synthetic or fully synthetic libraries or combinations thereof. Antibodies, antibody-based binding proteins and antigen-binding fragments of the invention include intact antibodies and antibody fragments or antigen-binding fragments that contain the antigen-binding portions of an intact antibody and retain the capacity to bind the cognate antigen. Unless otherwise specified herein, all peptide sequences, including all antibody and antigen-binding fragment sequences are referred to in N -> C order.

An intact antibody typically comprises at least two heavy (H) chains (about 50-70 kD) and two light (L) chains (about 25 kD) inter-connected by disulfide bonds. The recognized immunoglobulin genes encoding antibody chains include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Each heavy chain of an antibody is comprised of a heavy chain variable region ($V_H$) and a heavy chain constant region. In the case of IgG, the heavy chain constant region is comprised of three domains, $C_{H1}$, $C_{H2}$ and $C_{H3}$. Each light chain is comprised of a light chain variable region ($V_L$) and a light chain constant region. The light chain constant region is comprised of one domain, $C_L$. The variable regions of the heavy and light chains contain a binding domain that interacts with an antigen. The constant regions of the antibodies may mediate the binding of the immunoglobulin to host tissues or factors, including various cells of the immune system and the first component (Clq) of the classical complement system. Monoclonal antibodies (mAbs) consist of identical antibodies molecules.

The $V_H$ and $V_L$ regions of an antibody can be further subdivided into regions of hypervariability, also termed complementarity-determining regions (CDRs), which are interspersed with the more conserved framework regions (FRs). Each $V_H$ and $V_L$ is composed of three CDRs and four FRs, arranged from amino-terminus to carboxyl-terminus in the following order: FR1, CDR1, FR2, CDR2, FR3, CDR3, FR4. The locations of CDR and FR regions and a numbering system have been defined, e.g., the IMGT system (Lefranc MP et al., 2015), as used herein.

Antibodies, antibody-based binding proteins and antigen-binding fragments of the invention also encompass single chain antibodies. The term "single chain antibody" refers to a polypeptide comprising a $V_H$ domain and a $V_L$ domain in polypeptide linkage, generally linked via a spacer peptide, and which may comprise additional domains or amino acid sequences at the amino- and/or carboxyl-termini. For example, a single-chain antibody may comprise a tether segment for linking to the encoding polynucleotide. As an example, a single chain variable region fragment (scFv) is a single-chain antibody. Compared to the $V_L$ and $V_H$ domains of the Fv fragment that are coded for by separate genes, a scFv has the two domains joined (e.g., via recombinant methods) by a synthetic linker. This enables them to be made as a single protein chain in which the $V_L$ and $V_H$ regions pair to form monovalent molecules.

Examples of antibody-based binding proteins are polypeptides in which the binding domains of the antibodies are combined with other polypeptides or polypeptide domains, e.g. alternative molecular scaffolds, Fc-regions, other functional or binding domains of other polypeptides or antibodies resulting in molecules with addition binding properties, e.g. bi- or multispecific proteins or antibodies. Such polypeptides can create an arrangement of binding or functional domains normally not found in naturally occurring antibodies or antibody fragments.

Examples of antigen-binding fragments include (i) a Fab fragment, a monovalent fragment consisting of the $V_L$, $V_H$, $C_L$ and $C_{H1}$ domains; (ii) a $F(ab')_2$ fragment, a bivalent fragment comprising two Fab fragments linked by a disulfide bridge at the hinge region; (iii) a Fd fragment consisting of the $V_H$ and $C_{H1}$ domains; (iv) a Fv fragment consisting of the $V_L$ and $V_H$ domains of a single arm of an intact antibody; (v) disulfide stabilized Fvs (dsFvs) which have an interchain disulfide bond engineered between structurally conserved framework regions; (vi) a single domain antibody (dAb) which consists of a $V_H$ or $V_L$ domain (see, e.g., Ward et al., Nature 341:544-546, 1989); and (vii) an isolated

complementarity determining region (CDR) as a linear or cyclic peptide.

Antigen-binding fragments of the present invention also encompass single domain antigen-binding units that have a camelid scaffold. Animals in the camelid family include camels, llamas, and alpacas. Camelids produce functional antibodies devoid of light chains. The heavy chain variable ($V_H$) domain folds autonomously and functions independently as an antigen-binding unit. Its binding surface involves only three CDRs as compared to the six CDRs in classical antigen-binding molecules (Fabs) or single chain variable fragments (scFvs). Camelid antibodies are capable of attaining binding affinities comparable to those of conventional antibodies.

The terms "human antibody" refer to an antibody, antibody-based binding protein or antigen-binding fragment that contains sequences derived from human immunoglobulins such that substantially all of the CDR regions are of human origin, and substantially all of the FR regions correspond to those of a human immunoglobulin sequence.

The terms "alternative scaffold" and "antibody mimetic" refer to proteins not belonging to the immunoglobulin family, and even non-proteins such as aptamers, or synthetic polymers. Some types have an antibody-like beta-sheet structure. Potential advantages of "antibody mimetics" or "alternative scaffolds" over antibodies are better solubility, higher tissue penetration, higher stability towards heat and enzymes, and comparatively low production costs.

Some antibody mimetics can be provided in large libraries, which offer specific binding candidates against every conceivable target. Just like with antibodies, target specific antibody mimetics can be developed by use of High Throughput Screening (HTS) technologies as well as with established display technologies, just like phage display, bacterial display, yeast or mammalian display. Currently developed antibody mimetics encompass, for example, ankyrin repeat proteins (called DARPins), C-type lectins, A- domain proteins of S. aureus, transferrins, lipocalins, 10th type III domains of fibronectin, Kunitz domain protease inhibitors, ubiquitin derived binders (called affilins), gamma crystallin derived binders, cysteine knots or knottins, thioredoxin A scaffold based binders, nucleic acid aptamers, artificial antibodies produced by molecular imprinting of polymers, peptide libraries from bacterial genomes, SH-3 domains, stradobodies, "A domains" of membrane receptors stabilised by disulfide bonds and Ca2+, CTLA4-based compounds, Fyn SH3, and aptamers (oligonucleic acid or peptide molecules that bind to a specific target molecules)

## Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

All amino acid sequences disclosed herein are shown from N-terminus to C-terminus; all nucleic acid sequences disclosed herein are shown 5'->3'.

### Example 1. Generation of purified, recombinant anti-human ROR1 and isotype control antibodies

*Expression vectors:* Antibody variable region coding regions were produced by total gene synthesis (GenScript) using MNFGLRLIFLVLTLKGVQC as leader sequence, and were assembled with human IgH-γ1 and IgL-κ or IgL-λ constant regions, as applicable, in the expression vector pCB14. This vector, a derivative of the episomal mammalian expression vector pCEP4 (Invitrogen), carries the EBV replication origin, encodes the EBV nuclear antigen (EBNA-1) to permit extra-chromosomal replication, and contains a puromycin selection marker in place of the original hygromycin B resistance gene.

*Expression and purification*: pCB14-based expression vectors were transfected into HEK293T cells using Lipofectamine® LTX Reagent with PLUS™ Reagent (Thermo Fisher Scientific, Reinach, Switzerland, 15388100); following a 1-day incubation (37°C, 5% $CO_2$, growth media: Dulbecco's Modified Eagle Medium (DMEM) High Glucose (4.5g/L) with L-Glutamine with 10% (v/v) Fetal Calf Serum (FCS), 100IU/mL of Pen-Strep-Fungizone and 2mM L-glutamine (all Bioconcept, Allschwil, Switzerland)), cells were expanded under selection conditions (2μg/mL of puromycin (Sigma-Aldrich, Buchs SG, Switzerland, P8833-25mg stock at 2mg/mL)). Cells were split and further expanded (37°C, 5% $CO_2$); once confluency was reached, tissue culture dishes were coated with 20μg/ml poly-L-Lysine (Sigma-Aldrich, P1524) for 2h at 37°C and washed twice with PBS. Then, cells were trypsinized and split 1:3 onto poly-L-lysine-coated plates. Again after reaching confluency, cells were washed with PBS followed by media replacement to production media (DMEM/F-12, Gibco/Thermo Fisher Scientific, 31330-03) supplemented with 1μg/mL puromycin (Sigma, P8833), 100 IU/mL of Pen-Strep-Fungizone (Bioconcept), 161μg/mL of N-acetyl-L-cysteine (Sigma-Aldrich, A8199) and 10μg/mL of L-glutathione reduced (Sigma-Aldrich, G6529). Supernatant, harvested bi-weekly and filtered (0.22μm) to remove cells,

was stored at 4°C until purification.

For purification, filtered supernatant was loaded onto a PBS-equilibrated Protein A HiTrap column (GE Healthcare, Frankfurt am Main, Germany, 17-0405-01) or a JSR Amsphere™ Protein A column (JSR Life Sciences, Leuven, Belgium, JWT203CE) and washed with PBS; elution was performed using 0.1M glycine (pH 2.5) on an AEKTA pure (GE Healthcare). Fractions were immediately neutralized with 1M Tris-HCl buffer (pH 8.0), and analyzed for protein purity and integrity by SDS-PAGE. Protein-containing fractions were mixed and subjected to buffer exchange using Amicon filtration units (Millipore, Schaffhausen, Switzerland, UFC901008) to reach a dilution of 1:100 in PBS, and then sterile filtered using a low retention filter (0.20μm, Carl Roth, Karlsruhe, Germany, PA49.1).

Antibodies were transiently expressed in CHO cells by methods known in the art and recombinant antibodies were purified by standard protein A purification from CHO cell supernatants, as known in the art. The purity and the integrity of the recombinant antibodies were analyzed by SDS-PAGE.

**Table 1. Anti-hRORI and isotype control antibodies used in the Examples**

| **Antibody** | **Format** | **Antibody SEQ ID HC/LC** | **C-Terminal Tags (HC: Heavy Chain, LC: Light Chain)** |
|---|---|---|---|
| XBR1-402 (mAb202) | IgG | HC: SEQ ID NO.1<br>LC: SEQ ID NO.2 | HC: LPETG-Strep<br>LC: $G_4$LPETG-Strep |
| Ac10 (mAb046) | IgG | HC: SEQ ID NO.3<br>LC: SEQ ID NO.4 | HC: LPETG-Strep<br>LC: $G_4$SLPETG-Strep |
| 2A2 (mAb066) | IgG | HC: SEQ ID NO.5<br>LC: SEQ ID NO.6 | HC: LPETG-Strep<br>LC: $G_4$SLPETG-Strep |
| huXBR1-402-17 (mAb357) | IgG | HC: SEQ ID NO.20<br>LC: SEQ ID NO.21 | HC: none<br>LC: $G_4$SLPETG-TwinStrep |
| Ac10 (mAb340) | IgG | HC: SEQ ID NO.3<br>LC: SEQ ID NO.4 | HC: none<br>LC: $G_4$SLPETG-TwinStrep |

**Example 2. Conjugation of mAbs with glycine-modified toxins to form ADCs using SMAC-technology™**

*Sortase A.* A recombinant and affinity purified Sortase A enzyme based on the Sortase A of Staphylococcus aureus was produced in *E. coli* as per the techniques described in WO2014140317A1.

*Generation of glycine-modified toxins.* In order to generate SMAC-technology™ conjugated ADCs pentaglycine-EDA-PNU derivative (G5-EDA-PNU) was manufactured by Concortis (depicted in Figure 1). The identity and purity of the pentaglycine-modified toxins was confirmed by mass-spectrometry and HPLC, respectively. The G5-EDA-PNU exhibited >95% purity, as determined by HPLC chromatography.

*Sortase-mediated antibody conjugation.* The above-mentioned toxin was conjugated to antibodies as per Table 2 by incubating LPETG-tagged mAbs [10μM] with glycine modified toxin [200μM] and 3 μM Sortase A in the listed conjugation buffer for 3.5h at 25°C. The reaction was stopped by passing it through an rProtein A GraviTrap column (BioRad). Bound conjugate was eluted with 5 column volumes of elution buffer (0.1M glycine pH 2.5, 50nM NaCl), with 1 column volume fractions collected into tubes containing 25% v/v 1M HEPES pH 8 to neutralise the acid. Protein containing fractions were pooled and formulated in the formulation buffer of Table 2 using a ZebaSpin desalting column.

*ADC analytics.* Drug-antibody ratio (DAR) was assessed by Reverse Phase Chromatography performed on a Polymer Labs PLRP 2.1mm x 5cm, 5μm column run at 1mL/min/80°C with a 25-minute linear gradient between 0.05 and 0.1% TFA/$H_2O$ and 0.04 to 0.1% TFA/$CH_3CN$. Samples were first reduced by incubation with DTT at pH 8.0 at 37°C for 15 minutes. The DAR determined by Reverse Phase Chromatography is summarized in Table 2 below.

**Table 2: Analytical summary of ADCs manufactured in this study. DAR, drug-to-antibody ratio. ND, not determined.**

| **ADC** | **mAb** (ref.) | **Toxin** | **Conjugation Buffer** | **Formulation Buffer** | **DAR** |
|---|---|---|---|---|---|
| XBR1-402-G5-PNU | XBR1-402 (mAb202) | G5-PNU | 50mM HEPES (pH 7.5), 150mM NaCl, 5mM $CaCl_2$ | PBS | ND |

(continued)

| ADC | **mAb** (ref.) | Toxin | Conjugation Buffer | Formulation Buffer | DAR |
|---|---|---|---|---|---|
| Ac10-G5-PNU | Ac10 (mAb046) | G5-PNU | 50mM HEPES (pH 7.5), 150mM NaCl, 1mM $CaCl_2$ | PBS | 3.7 |
| Ac10-G3-PNU | Ac10 (mAb340) | G3-PNU | 250mM HEPES, pH 7.5, 5mM $CaCl_2$, 50% Glycerol | PBS | 1.9 |
| huXBR1-402-17-G3-PNU | huXBR1-402-17 | G3-PNU | 250mM HEPES, pH 7.5, 5mM $CaCl_2$, 50% Glycerol | 15mM Histidine, pH 6.5, 175mM Sucrose, 0.02% Tween20 | 1.9 |
| huXBR1-402-17-G2-PNU | huXBR1-402-17 | G2-PNU | 250mM HEPES, pH 7.5, 5mM $CaCl_2$, 50% Glycerol | 15mM Histidine pH 6.5, 175mM Sucrose, 0.02% Tween20 | 2.0 |

From these analyses it can be concluded that the SMAC-technology™ conjugation has proceeded at high efficiency.

**Example 3. Generation of EMT-6 cells stably expressing human ROR1**

Murine EMT-6 breast cancer cells were cultured in DMEM complete (Dulbecco's Modified Eagle Medium (DMEM) High Glucose (4.5g/L) with L-Glutamine with 10% (v/v) Fetal Calf Serum (FCS), 100 IU/mL of Pen-Strep-Fungizone and 2mM L-glutamine (all Bioconcept, Allschwil, Switzerland)) at 37°C and 5% $CO_2$. Cells were engineered to overexpress ROR1 by transposition as follows: cells were centrifuged (6min, 1200rpm, 4°C) and resuspended in RPMI-1640 media (5x$10^6$ cells/mL). 400μL of cell suspension was then added to 400μL of RPMI containing 13.3μg of either transposable vector pPB-PGK-Puro-ROR1 (directing co-expression of full-length ROR1 (NP_005003.2) along with the puromycin-resistance gene), and 6.6μg of transposase-containing vector pCDNA3.1_hy_mPB. DNA/EMT-6 cell mixture was transferred to electroporation cuvettes (0.4 cm-gap, 165-2088, BioRad, Cressier, Switzerland) and electroporated using the Biorad Gene Pulser II with capacitance extender at 300V and 950μF. Then, cells were incubated for 5-10min at room temperature. Following the incubation, cells were centrifuged at 1200rpm for 6min, washed once and subsequently resuspended in DMEM complete prior to incubation at 37°C in a humidified incubator at 5% $CO_2$ atmosphere. One day after electroporation, cell pools stably expressing human ROR1 were selected by adding 3 μg/mL puromycin (Sigma-Aldrich, P8833).

Single-cell clones expressing ROR1 were derived from antibiotic-selected EMT-6-ROR1 cells. Briefly, following trypsinization, $10^6$ cells were centrifuged in FACS tubes; obtained pellets were resuspended in buffer (PBS with 2% (v/v) FCS). Cells were then incubated with anti-RORl antibody 2A2 for 30min (4°C, final concentration 2μg/mL), followed by centrifugation and washing. Cells were then resuspended as previously and incubated with anti-human IgG antibody (Fc gamma-specific) PE (eBioscience, Vienna, Austria, 12-4998-82) with a 1:250 dilution in the dark (30min, 4°C), washed once in buffer and kept on ice until single-cell sorting of antigen-expressing cells by FACS using a FACSAriaII instrument (BD Biocsiences, San Jose, USA).

**Example 4. *In vivo* evaluation of anti-RORl ADCs in EMT-6-ROR1 syngeneic breast tumor model**

1x$10^6$ EMT-6-ROR1 clone 14 tumor cells in 100μl PBS were orthotopically implanted into the mammary fat pad of each BALB/c mouse. On reaching a mean tumor volume of approx. 30-80 $mm^3$ (by caliper), on Day 0 (D0), mice were block-randomized into groups of 8 animals each according to tumour size. Mice were then treated once with different doses (0.25 mg/kg, 0.5 mg/kg and 1 mg/kg) (Fig. 3), in order to determine a sub-optimal dose, in which the ADCs only result in a partial anti-tumor response. Following this, the same models were set up for treatment with the low dose ADC, including an isotype-matched control ADC and either with or without combination treatment with 10 mg/kg anti-CTLA4 immune checkpoint inhibitor mAb 9D9 according to Table 3. Mice were monitored up to at least 40 days, and euthanized in case of excessive tumor burden or signs of distress. Immune checkpoint inhibitor 9D9 is an inhibitor of mouse CTLA-4 and was formulated in PBS and was purchased at BioXcell, West Lebanon, New Hampshire, U.S.

**Table 3: Experimental groups for *in vivo* evaluation of PNU toxin-based ADCs in combination with checkpoint inhibitors**

| **Group** | | **Treatment concentration** | **Application** | | |
|---|---|---|---|---|---|
| | | | Volume | Route | Scheme after randomization |
| **1** | Vehicle Control | - | 5 ml/kg | i.v. | Once D0 |
| **2** | Ac10-G5-PNU (isotype control) | 0.25 mg/kg | 5 ml/kg | i.v. | Once D0 |
| **4** | XBR1-402-G5-PNU | 0.25 mg/kg | 5 ml/kg | i.v. | Once D0 |
| **6** | α-mCTLA-4 antibody (9D9)<br>+<br>Ac10-G5-PNU (isotype control) | 10 mg/kg<br>+<br>0.25 mg/kg | 5 ml/kg | i.p.<br>+<br>i.v. | D2 and two further treatments 3-4 days apart<br>+<br>Once D0 |
| **11** | α-mCTLA-4 antibody (9D9)<br>+<br>XBR1-402-G5-PNU | 10 mg/kg<br>+<br>0.25 mg/kg | 5 ml/kg<br>+<br>5 ml/kg | i.p.<br>+<br>i.v. | D2 and two further treatments 3-4 days apart<br>+<br>Once D0 |

Tumor volumes were determined based on twice-weekly caliper measurement of tumor size. For calculation of the group median tumor volumes, the values from animals that were alive on the day in question were considered. In addition, tumor volumes of animals that were euthanized due to their tumor load were carried forward using the Last-Observation-Carried-Forward (LOCF) methodology for as long as this increased the group mean/median tumor volume.

Figure 4 presents tumor volume evolution for individual mice, as well as median tumor volume evolution. Tumor volume evolution for individual mice treated with (A) vehicle control, (B) Ac10-G5-PNU isotype control, (C) XBR1-402-G5-PNU, (D) Ac10-G5-PNU isotype control plus anti-mouse CTLA-4 antibody 9D9, (E) XBR1-402-G5-PNU plus anti-mouse CTLA-4 antibody 9D9. (F) Median group tumor volume evolution (with application of Last-Observation-Carried-Forward (LOCF) methodology) for the following groups: vehicle control, Ac10-G5-PNU isotype control and XBR1-402-G5-PNU. (G) Median group tumor volume evolution (with application of Last-Observation-Carried-Forward (LOCF) methodology) for the following groups: vehicle control, Ac10-G5-PNU isotype control and anti-mouse CTLA-4 antibody 9D9, and XBR1-402-G5-PNU and anti-mouse CTLA-4 antibody 9D9.

As shown by the results in the figure, combination of XBR1-402-G5-PNU and anti-mouse CTLA-4 antibody 9D9 provides enhanced anti-tumor responses relative to the individual treatment with ADC at 0.25 mg/kg alone. Further, panel D shows that, relative to vehicle (Panel A) and isotype controls (Panel B), the anti-mouse CTLA-4 antibody 9D9 shows some anti-tumor efficacy, supporting that the EMT-6-ROR1 tumor model can be characterized as a hot tumor model.

Additionally, all surviving mice in group 11 were protected from tumor growth on re-challenge (day 102) with $1x10^6$ EMT-6-ROR1 clone 14 tumor cells, implanted in the mammary pad having no prior injection (not shown).

**Example 5. Generation of CT-26 and B16-F10 cells stably expressing human ROR1**

Murine CT-26 colon carcinoma cells and B16-F10 ("B16") melanoma cells (both from the ATCC) were cultured in RPMI media with 10% (v/v) Fetal Calf Serum (FCS), 100 IU/mL of Pen-Strep-Fungizone and 2 mM L-glutamine (all Bioconcept, Allschwil, Switzerland) at 37°C and 5% $CO_2$. Cells were engineered to overexpress ROR1 by transposition as follows: cells were centrifuged (6min, 1200rpm, 4°C) and resuspended in RPMI media ($5x10^6$ cells/mL). 400μL of cell suspension was then added to 400μL of RPMI containing 13.3μg of transposable vector pPB-PGK-Puro-ROR1 (directing co-expression of full-length ROR1 (NP_005003.2) along with the puromycin-resistance gene), and 6.6μg of transposase-containing vector pcDNA3.1_hy_mPB. The DNA/CT-26 or B16 cell mixture was transferred to electroporation cuvettes (0.4 cm-gap, 165-2088, BioRad, Cressier, Switzerland) and electroporated using the Biorad Gene Pulser II with capacitance extender at 300V and 950μF. Then, cells were incubated for 5-10min at room temperature. Following the incubation, cells were centrifuged at 1200rpm for 6min, washed once and subsequently resuspended in RPMI complete prior to incubation at 37°C in a humidified incubator at 5% $CO_2$ atmosphere. One day after electroporation, cell pools stably expressing human ROR1 were selected by adding 3 μg/mL puromycin (Sigma-Aldrich, P8833).

Single-cell clones of CT-26 expressing ROR1 were derived from antibiotic-selected cells. Briefly, following

trypsinization, $10^6$ cells were centrifuged in FACS tubes; obtained pellets were resuspended in buffer (PBS with 2% (v/v) FCS). Cells were then incubated with anti-ROR1 antibody XBR1-402 for 30 min (4°C, final concentration 2 μg/mL), followed by centrifugation and washing. Cells were then resuspended as previously and incubated with anti-human IgG antibody (Fc gamma-specific) PE (eBioscience, Vienna, Austria, 12-4998-82) at a 1:250 dilution in the dark (30min, 4°C), washed once in buffer and kept on ice until single-cell sorting of antigen-expressing cells by FACS using a FACSAria II instrument (BD Biocsiences, San Jose, USA). Figure 5 (A) shows the FACS staining results of the engineered CT-26 clone 3. Expression of ROR1 on the B16 pools used in the experiment below was also determined by FACS (Figure 5 (B)).

**Example 6. hRORI-overexpressing CT26 colon carcinoma mouse model treated with anti-RORI ADCs and combinations with immune checkpoint inhibitors**

CT26 establishes warm-to-hot tumors in mouse models, meaning that these respond to immune checkpoint inhibitors when dosed sufficiently high (Mosely, S. et al., 2017). Experimental groups, as per Table 4, each contained eight 6-8-week-old female BALB/c mice. 1x$10^6$ CT-26-ROR1 clone 3 tumor cells (from Example 5) in 100 μl PBS were implanted unilaterally into the flank of each mouse. On reaching a mean tumor volume of approx. 30-100 $mm^3$ (by caliper), on Day 0 (D0), mice were randomized into groups according to tumour size. Mice were then treated according to Table 4 and monitored three times per week for 42 days or until the tumors reached 1500 $mm^3$ (by caliper). RPM1-14 (BioXcell, West Lebanon, New Hampshire, U.S) is an inhibitor of mouse PD-1 and was formulated in PBS. This immune checkpoint inhibitor was dosed below its effective dose as a monotherapy, as previously determined by Oncotest (data not shown).

**Table 4: Experimental groups for *in vivo* evaluation of anti-RORI ADCs with immune checkpoint inhibitors**

| **Group (group size)** | | **Treatment concentration** | **Application** | |
|---|---|---|---|---|
| | | | Route | Scheme after randomization |
| **1** | Vehicle Control | - | i.v.<br>i.p. | Once D0<br>D0, D4, D8 |
| **2** | Ac10-G3-PNU (isotype) | 4 mg/kg | i.v. | Once D0 |
| **3** | huXBR1-402-17-G3-PNU | 4 mg/kg | i.v. | Once D0 |
| **4** | Ac10-G3-PNU<br>+<br>α-mPD-1 antibody (RPM1-14) | 4 mg/kg<br>+<br>5 mg/kg | i.v.<br>+<br>i.p. | Once D0<br>+<br>D0, D4, D8 |
| **5** | huXBR1-402-17-G3-PNU<br>+<br>α-mPD-1 antibody (RPM1-14) | 4 mg/kg<br>+<br>5 mg/kg | i.v.<br>+<br>i.p. | Once D0<br>+<br>D0, D4, D8 |

Figure 6 presents tumor volume evolution for individual mice in each group: (A) vehicle control (untreated) of group 1, (B) isotype control of group 2, (C) anti-RORI ADC huXBR1-402-17-G3-PNU of group 3, (D) isotype control plus immune checkpoint inhibitor anti-mouse PD-1 antibody of group 4, (E) anti-RORI ADC huXBR1-402-17-G3-PNU plus immune checkpoint inhibitor anti-mouse PD-1 antibody of group 7. It is underlined that the targeted-ADC was dosed at a dose significantly lower than required for full response as monotherapy, as shown in Panel C. The results suggest a strong synergy between the evaluated immune checkpoint inhibitor and the targeted PNU derivative-comprising ADC.

**Example 7. hRORI-overexpressing B16 melanoma mouse model treated with anti-ROR1 ADCs and combinations with immune checkpoint inhibitors**

B16 establishes poorly immunogenic (cold) tumors in mouse models (Celik, C. et al. 1983), meaning that these do not respond to immune checkpoint inhibitors when dosed within a reasonable range, i.e., up to about 50 to 100μg/mouse (Grosso, J. et al., 2013). Experimental groups, as per Table 7, each contained ten 8-9-week-old female BL6/6NRj. 2x$10^5$ B16-ROR1 tumor cells (from Example 5) in 100μl of Hanks' Balanced Salt solution (HBSS, Thermo Fischer) were injected i.v. into each mouse at Day 0 (D0). Mice were randomized into groups by simple random allocation

and treated according to Table 5, with monitoring 5 days a week over 12-19 days.

**Table 5: Experimental groups for *in vivo* evaluation of anti-RORI ADCs with immune checkpoint inhibitors**

| Group (group size) | | Treatment concentration | Application | |
|---|---|---|---|---|
| | | | Route | Scheme after randomization |
| **1** | Vehicle Control | - | i.v.<br>i.p. | Once D1<br>D1, D5, D9 |
| **2** | huXBR1-402-17-G2-PNU | 4 mg/kg | i.v. | Once D1 |
| **3** | α-mCTLA-4 antibody (9D9) | 10 mg/kg | i.p. | D1, D5, D9 |
| **4** | huXBR1-402-17-G2-PNU<br>+<br>α-mCTLA-4 antibody (9D9) | 4 mg/kg<br>+<br>10 mg/kg | i.v.<br><br>i.p. | Once D1<br>+<br>D1, D5, D9 |

At the last day of monitoring, mice were euthanized by $CO_2$ followed by whole-lung collection and fixation in Bouin's solution (Labforce Bio-Optica). Experimentally induced metastasis (Saxena M. et al., 2013) on each lung surface (representing B16-ROR1 colonies) were visually counted and each was assessed for assignment to one of three bins (diameter <1mm, 1-2mm, >2mm) under blinded conditions. The volume of the colonies on each lung was then determined according to the following formula (approximating each colony as a sphere):

$$\text{Colony volume (mm}^3\text{)} = \text{count (spots with diameter<1mm)} \times 0.004\text{mm}^3 + \text{count (spots with diameter 1-2mm)} \times 0.21\text{mm}^3 + \text{count(spots with diameter>2mm)} \times 0.485\text{mm}^3$$

Statistical analyses were performed with GraphPad Prism and unpaired T-test of average. A p-value of <0.05 was considered statistically significant (*), p-value of ≥0.5 was considered not statistically significant (n.s.).

Figure 7 presents the colony volumes of the different treatment groups of Table 5. These results suggest, in a poorly immunogenic ("cold") tumor, a strong synergy between the evaluated immune checkpoint inhibitor and the targeted PNU derivative-comprising ADC.

**Example 8. hRORI-overexpressing B16 melanoma mouse model treated with anti-ROR1 ADCs**

Experimental groups, as per Table 8, contained 8-9-week-old female BL6/6NRj. $10^6$ B16-ROR1 tumor cells (from Example 5) in 100μl of Hanks' Balanced Salt solution (HBSS, Thermo Fischer) were implanted unilaterally into the flank each mouse. On reaching a mean tumor volume of approx. about 250 $mm^3$ (by caliper), on Day 0 (D0), mice were randomized into groups according to tumour size as per Table 6. Mice were then treated according to Table 6 and were euthanized 24-hours after administration, followed by tumor extraction.

**Table 6: Experimental groups for *in vivo* evaluation of anti-RORI ADC**

| Group (group size) | | Treatment concentration | Mice in group | Administra tion Route | Treatment Schedule |
|---|---|---|---|---|---|
| | | | | | |
| **1** | Untreated control | - | 1 | - | - |
| **2** | huXBR1-402-17-G2-PNU | 4 mg/kg | 3 | i.v. | D0 |

Tumors were snap-frozen in optimal cutting temperature compound (oct, from Fischer Scientific). Cryo-sections were stained using primary commercial anti-mouse CD4 and anti-mouse CD8 antibodies (GK1.5 BioLegend 100402, and 53-6.7 BioLegend 100702, respectively). Detection was performed using an anti-rat antibody (AlexaFluor488, Invitrogen A21208).

Figure 8 shows representative images of CD4 and CD8-staining of hRORl-overexpressing B16 tumor cryo-sections from (A) the untreated control mouse, and (B) the three mice treated with ADCs (once, at 4 mg/kg) according to Table 6. As per Figure 8(A), B16 tumors naturally contains low levels of CD4+ and CD8+ cells, in keeping with the status of B16 tumors as cold tumors. Figure 8(B) shows that treatment with the ADC, as per the present invention, increases the number of CD4+ and CD8+ cells within the tumor.

## REFERENCES

Galon J et al., J Pathol 2014; 232: 199-209

Pages F et al., J Clin Oncol 2009; 27: 5944-5951.

Angell HK, Galon J, Curr Opin Immunol 2013; 25: 261-267.

Galon et al. Immunity 2013; 39: 11-26.

Bu X et al., Trends in Molecular Medicine (2016), Volume 22, Issue 6, 448 - 451

Shi et al., Org. Biomol. Chem., 2009,7, 3709-3722

Mosely, S. et al. Cancer Immunology Research, January 2017

Saxena M and Christofori G., Molecular Oncology 7, 2013, 283-296

Celik, C. et al. Cancer Res 43, 3507-3510, 1983

Grosso, J. et al. Cancer Immunity, 22, 13(5), 2013


## SEQUENCES

The following sequences form part of the disclosure of the present application. A WIPO ST 25 compatible electronic sequence listing is provided with this application, too. For the avoidance of doubt, if discrepancies exist between the sequences in the following table and the electronic sequence listing, the sequences in this table shall be deemed to be the correct ones.

| | | |
|---|---|---|
| 1 | XBR1-402 HC amino acid sequence | QEQQKESGGGLFKPTDTLTLTCTASGFDISSYYMSWVRQAPGNGLEWIGAIGISGNAYY<br>ASWAKSRSTITRNTNLNTVTLKMTSLTAADTATYFCARDHPTYGMDLWGPGTLVTVSSA<br>STKGPSVFPLAPSSKSTSGGTAALGCLVKDYFPEPVTVSWNSGALTSGVHTFPAVLQSS<br>GLYSLSSVVTVPSSSLGTQTYICNVNHKPSNTKVDKKVEPKSCDKTHTCPPCPAPELLG<br>GPSVFLFPPKPKDTLMISRTPEVTCVVVDVSHEDPEVKFNWYVDGVEVHNAKTKPREEQ<br>YNSTYRVVSVLTVLHQDWLNGKEYKCKVSNKALPAPIEKTISKAKGQPREPQVYTLPPS<br>RDELTKNQVSLTCLVKGFYPSDIAVEWESNGQPENNYKTTPPVLDSDGSFFLYSKLTVD<br>KSRWQQGNVFSCSVMHEALHNHYTQKSLSLSPGK |
| 2 | XBR1-402 LC amino acid sequence | SYELTQLPSVSVSLGQTARITCEGNNIGSKAVHWYQQKPGLAPGLLIYDDDERPSGVPD<br>RFSGSNSGDTATLTISGAQAGDEADYYCQVWDSSAYVFGGGTQLTVTGQPKAAPSVTLF<br>PPSSEELQANKATLVCLISDFYPGAVTVAWKADSSPVKAGVETTTPSKQSNNKYAASSY<br>LSLTPEQWKSHKSYSCQVTHEGSTVEKTVAPTECS |
| 3 | AclO HC amino acid sequence | QIQLQQSGPEVVKPGASVKISCKASGYTFTDYYITWVKQKPGQGLEWIGWIYPGSGNTK<br>YNEKFKGKATLTVDTSSSTAFMQLSSLTSEDTAVYFCANYGNYWFAYWGQGTQVTVSAA<br>STKGPSVFPLAPSSKSTSGGTAALGCLVKDYFPEPVTVSWNSGALTSGVHTFPAVLQSS<br>GLYSLSSVVTVPSSSLGTQTYICNVNHKPSNTKVDKKVEPKSCDKTHTCPPCPAPELLG<br>GPSVFLFPPKPKDTLMISRTPEVTCVVVDVSHEDPEVKFNWYVDGVEVHNAKTKPREEQ<br>YNSTYRVVSVLTVLHQDWLNGKEYKCKVSNKALPAPIEKTISKAKGQPREPQVYTLPPS<br>RDELTKNQVSLTCLVKGFYPSDIAVEWESNGQPENNYKTTPPVLDSDGSFFLYSKLTVD<br>KSRWQQGNVFSCSVMHEALHNHYTQKSLSLSPGK |
| 4 | Ac10 LC amino acid sequence | DIVLTQSPASLAVSLGQRATISCKASQSVDFDGDSYMNWYQQKPGQPPKVLIYAASNLE<br>SGIPARFSGSGSGTDFTLNIHPVEEEDAATYYCQQSNEDPWTFGGGTKLEIKRTVAAPS<br>VFIFPPSDEQLKSGTASVVCLLNNFYPREAKVQWKVDNALQSGNSQESVTEQDSKDSTY<br>SLSSTLTLSKADYEKHKVYACEVTHQGLSSPVTKSFNRGEC |
| 5 | 2A2 HC amino acid sequence | QVQLQQSGAELVRPGASVTLSCKASGYTFSDYEMHWVIQTPVHGLEWIGAIDPETGGTA<br>YNQKFKGKAILTADKSSSTAYMELRSLTSEDSAVYYCTGYYDYDSFTYWGQGTLVTVSA<br>ASTKGPSVFPLAPSSKSTSGGTAALGCLVKDYFPEPVTVSWNSGALTSGVHTFPAVLQS<br>SGLYSLSSVVTVPSSSLGTQTYICNVNHKPSNTKVDKKVEPKSCDKTHTCPPCPAPELL<br>GGPSVFLFPPKPKDTLMISRTPEVTCVVVDVSHEDPEVKFNWYVDGVEVHNAKTKPREE<br>QYNSTYRVVSVLTVLHQDWLNGKEYKCKVSNKALPAPIEKTISKAKGQPREPQVYTLPP<br>SRDELTKNQVSLTCLVKGFYPSDIAVEWESNGQPENNYKTTPPVLDSDGSFFLYSKLTV<br>DKSRWQQGNVFSCSVMHEALHNHYTQKSLSLSPGK |
| 6 | 2A2 LC amino acid sequence | DIVMTQSQKIMSTTVGDRVSITCKASQNVDAAVAWYQQKPGQSPKLLIYSASNRYTGVP<br>DRFTGSGSGTDFTLTISNMQSEDLADYFCQQYDIYPYTFGGGTKLEIKRTVAAPSVFIF<br>PPSDEQLKSGTASVVCLLNNFYPREAKVQWKVDNALQSGNSQESVTEQDSKDSTYSLSS<br>TLTLSKADYEKHKVYACEVTHQGLSSPVTKSFNRGEC |

(continued)

| | | |
|---|---|---|
| 7 | Staphylococcus aureus sortase A recognition sequence 1, with X being any amino acid | -LPXTG |
| 8 | Staphylococcus aureus sortase A recognition sequence 2, with X being any amino acid | -LPXAG |
| 9 | recognition sequence for Staphylococcus aureus sortase A or engineered sortase A 4S-9 from Staphylococcus aureus, with X being any amino acid | -LPXSG |
| 10 | recognition sequence for engineered sortase A 2A-9 from Staphylococcus aureus, with X being any amino acid | -LAXTG |
| 11 | Streptococcus pyogenes sortase A recognition sequence, with X being any amino acid | -LPXTA |
| 12 | Staphylococcus aureus sortase recognition sequence | -NPQTN |
| 13 | Linker derived from Staphylococcus aureus sortase A recognition sequence 1, with X being any amino acid and n ≥ 1 and ≤ 21 | -LPXT(Gn)- |
| 14 | Linker derived from Staphylococcus aureus sortase A recognition sequence 2, with X being any amino acid and n ≥ 1 and ≤ 21 | -LPXA(Gn)- |
| 15 | Linker derived from recognition sequence for Staphylococcus aureus sortase A or engineered sortase A 4S-9 from Staphylococcus aureus, with X being any amino acid and n ≥ 1 and ≤ 21 | -LPXS(Gn)- |
| 16 | Linker derived from recognition sequence for engineered sortase A 2A-9 from Staphylococcus aureus, with X being any amino acid and n ≥ 1 and ≤ 21 | -LAXT(Gn)- |
| 17 | Linker derived from Streptococcus pyogenes sortase A recognition sequence, with X being any amino acid and n ≥ 1 and ≤ 21 | -LPXT(Gn)- or -LPXT(An)- |
| 18 | Linker derived from Staphylococcus aureus sortase recognition sequence, with n ≥ 1 and ≤ 21 | -NPQT (Gn) - |
| 19 | leader sequence | MNFGLRLIFLVLT LKGVQC |
| 20 | huXBR1-402-17 HC amino acid sequence | QVQLRESGPGLVKPSETLSLTCTVSGFDISSYYMSWVRQPPGKGLEWIGAIGISGNAYY<br>ASWAKSRVTISRDTSKNQFSLKLSSVTAADTAVYYCARDHPTYGMDLWGPGTLVTVSSA<br>STKGPSVFPLAPSSKSTSGGTAALGCLVKDYFPEPVTVSWNSGALTSGVHTFPAVLQSS<br>GLYSLSSVVTVPSSSLGTQTYICNVNHKPSNTKVDKKVEPKSCDKTHTCPPCPAPELLG<br>GPSVFLFPPKPKDTLMISRTPEVTCVVVDVSHEDPEVKFNWYVDGVEVHNAKTKPREEQ<br>YNSTYRVVSVLTVLHQDWLNGKEYKCKVSNKALPAPIEKTISKAKGQPREPQVYTLPPS<br>RDELTKNQVSLTCLVKGFYPSDIAVEWESNGQPENNYKTTPPVLDSDGSFFLYSKLTVD<br>KSRWQQGNVFSCSVMHEALHNHYTQKSLSLSPGK |
| 21 | huXBR1-402-17 LC amino acid sequence | SYELTQPPSVSVAPGKTARITCEGNNIGSKAVHWYQQKPGQAPVLVIYDDDERPSGIPE<br>RFSGSNSGNTATLTISRVEAGDEADYYCQVWDSSAYVFGGGTKLTVLGQPKAAPSVTLF<br>PPSSEELQANKATLVCLISDFYPGAVTVAWKADSSPVKAGVETTTPSKQSNNKYAASSY<br>LSLTPEQWKSHRSYSCQVTHEGSTVEKTVAPTECS |

## Claims

1. A binding protein-toxin conjugate comprising one or more anthracycline toxin moieties conjugated to a binding protein (for the manufacture of a medicament) for the treatment of a patient

    - suffering from,
    - being at risk of developing, and/or
    - being diagnosed for

    a neoplastic disease characterized as being a cold tumor.

2. A method of treating or preventing a neoplastic disease characterized as being a cold tumor, said method comprising administering to a patient a binding protein-toxin conjugate comprising one or more anthracycline toxin moieties.

3. A combination of

    (i) a binding protein-toxin conjugate comprising one or more anthracycline toxin moieties, conjugated to a binding protein, and
    (ii) an immune checkpoint inhibitor

    (for the manufacture of a medicament) for the treatment of a patient

    - suffering from,
    - being at risk of developing, and/or
    - being diagnosed for

    a neoplastic disease characterized as being a cold tumor.
    wherein the binding protein-toxin conjugate and the immune checkpoint inhibitor are administered to the patient simultaneously or sequentially, in any order.

4. A method of treating or preventing a neoplastic disease characterized as being a cold tumor, said method comprising administering to a patient

    (i) a binding protein-toxin conjugate comprising one or more anthracycline toxin moieties, conjugated to a binding protein, and
    (ii) an immune checkpoint inhibitor

    wherein the binding protein-toxin conjugate and the immune checkpoint inhibitor are administered to the patient

simultaneously or sequentially, in any order..

5. The binding protein-toxin conjugate or method according to claim 1 or 2, or combination according to claim 3 or 4, wherein the neoplastic disease characterized as being a cold tumor characterized has having an immunoscore of < 1.

6. The binding protein-toxin conjugate or method or combination according to any one of claims 1 - 5 wherein at least one anthracycline toxin moiety is a derivative of the anthracycline PNU-159682 having the following formula (i)

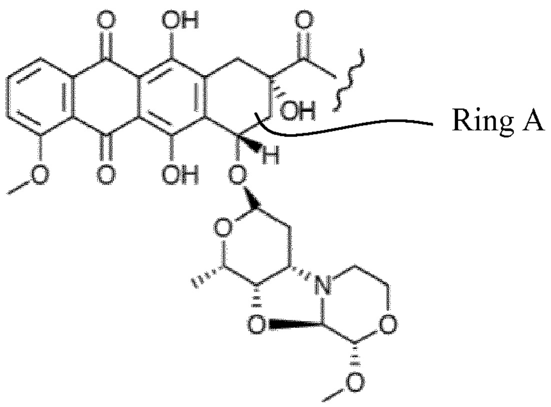

formula (i)

said toxin being conjugated at its wavy line to the binding protein via a linker.

7. The combination or method according to any one of claims 3 - 6, wherein the Immune Checkpoint inhibitor is at least one selected from the group consisting of

- anti PD-1
- anti CTLA-4
- anti PD-L1
- anti LAG3
- anti TIM3, anti OX40/OX40L (CD134/CD134L)
- anti CD112
- anti CD155
- anti B7-H3
- anti B7-H4
- anti IDO1
- anti IDO2
- anti TDO2
- anti TIGIT, and/or
- anti Galectin-9.

8. The binding protein-toxin conjugate or method or combination according to any one of claims 1 - 7, said conjugate comprising at its wavy line a linker structure X - $L_1$ - $L_2$ - $L_3$ - Y, wherein $L_1$ - $L_3$ represent linkers, and two of $L_1$ - $L_3$ are mandatory, and wherein X and Y further represent each one or more optional linkers.

9. The binding protein-toxin conjugate or method or combination according to claim 8, wherein the linker structure comprises, as $L_2$, an oligo-glycine peptide $(Gly)_n$ coupled to said anthracycline derivative, directly or by means of another linker $L_1$, in such a way that the oligo-glycine $(Gly)_n$ peptide has a free amino terminus, and wherein n is an integer $\geq 1$ and $\leq 21$.

10. The binding protein-toxin conjugate or method or combination according to claim 9, wherein the oligo-glycine peptide $(Gly)_n$ is conjugated to the anthracycline derivative of formula (i) by means of an alkylenediamino linker (EDA), designated as $L_1$, which alkylenediamino linker is conjugated to the anthracycline derivative by means of a first amide bond, while it is conjugated to the carboxy terminus of the oligo-glycine peptide by means of a second amide

bond, said conjugate of alkylenediamino linker and oligo-glycine peptide having the following formula (ii),

$$\text{\{}-NH-(CH_2)_m-NH-(Gly)_n-NH_2 \qquad \text{formula (ii)}$$

wherein the wavy line indicates the linkage to the anthracycline derivative of formula (i), wherein m is an integer ≥ 1 and ≤ 11 and n is an integer ≥ 1 and ≤ 21.

**11.** The binding protein-toxin conjugate or method or combination according to claim 9 or 10, wherein the oligo-glycine peptide $(Gly)_n$ is, directly or by means of another linker $L_1$, coupled to Ring A of the anthracycline derivative of formula (ii).

**12.** The binding protein-toxin conjugate or method or combination according to claim 9, wherein the oligo-glycine peptide $(Gly_n)$ is conjugated to the anthracycline derivative of formula (ii) by means of an alkyleneamino linker (EA), designated as $L_1$, which alkyleneamino linker is conjugated to the carboxy terminus of the oligo-glycine peptide by means of an amide bond, said conjugate of alkyleneamino linker and oligo-glycine peptide having the following formula (iii)

$$\text{\{}-(CH_2)_m-NH-(Gly)_n-NH_2 \qquad \text{formula (iii)}$$

wherein the wavy line indicates the linkage to the anthracycline derivative of formula (ii), wherein m is an integer ≥ 1 and ≤ 11 and n is an integer between ≥ 1 and ≤ 21.

**13.** The binding protein-toxin conjugate or method or combination according to any one of claims 8 - 12, wherein the linker structure $L_3$ comprises a peptide motif that results from specific cleavage of a sortase enzyme recognition motif.

**14.** The binding protein-toxin conjugate or method or combination according to claim 13, wherein said sortase enzyme recognition motif comprises at least one of the following amino acid sequences: LPXTG, LPXAG, LPXSG, LAXTG, LPXTA or NPQTN, with X being any conceivable amino acid sequence.

**15.** The binding protein-toxin conjugate or method or combination according to claim 13 or 14, wherein the resulting linker has at least one of the follwing amino acid sequences: - LPXTGn-, -LPXAGn-, -LPXSGn-, -LAXTGn-, -LPXTGn-, -LPXTAn-or -NPQTGn-, with Gn being an oligo- or polyglycine with n being an integer between ≥ 1 and ≤ 21, An being an oligo-or polyalanine with n being an integer between ≥ 1 and ≤ 21, and X being any conceivable amino acid sequence.

**16.** The binding protein-toxin conjugate or method or combination according to any one of claims 6 - 15, wherein the anthracycline derivative is conjugated, by means of the one or more linkers, to the carboxy termius of the binding protein, or to the carboxy terminus of at least one domain or subunit thereof.

**17.** The binding protein-toxin conjugate or method or combination according to any one of claims 6 - 16, wherein the binding protein is conjugated to the free amino terminus of the oligo-glycine peptide $(Gly_n)$ by means of an amide bond.

**18.** The binding protein-toxin conjugate or method or combination according to any one of claims 6 - 17, wherein the binding protein is at least one selected from the group consisting of an antibody, an antibody-based binding protein, a modified antibody format retaining target binding capacity, an antibody derivative or a fragment retaining target binding capacity, an alternative scaffold and/or an antibody mimetic.

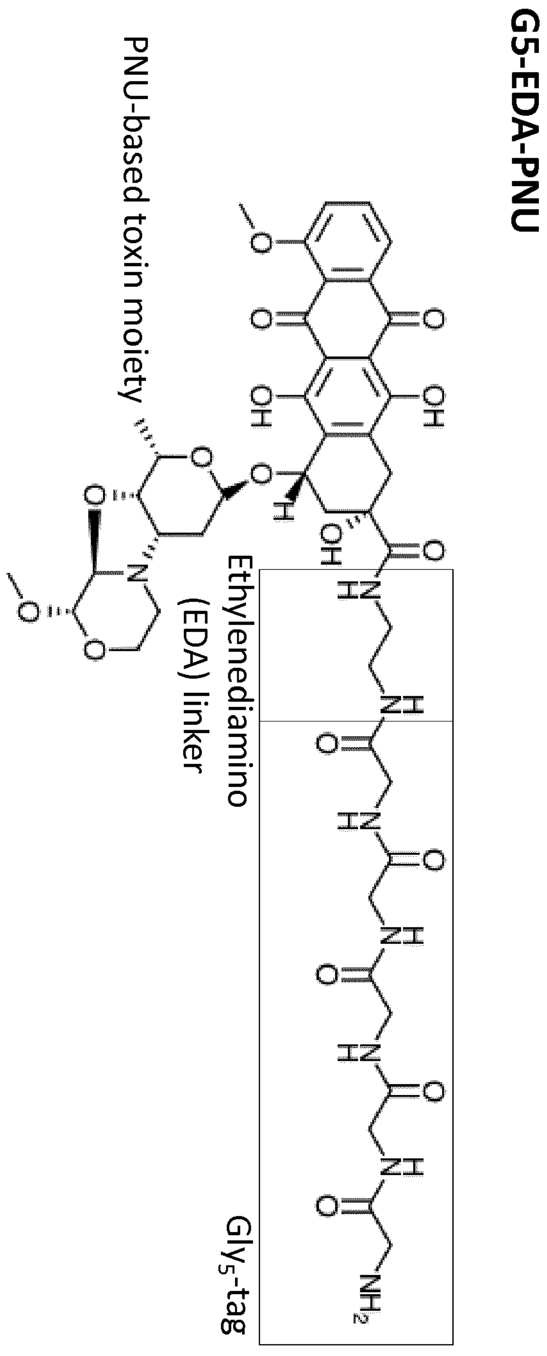
G5-EDA-PNU
PNU-based toxin moiety
Ethylenediamino (EDA) linker
$Gly_5$-tag

Fig. 1

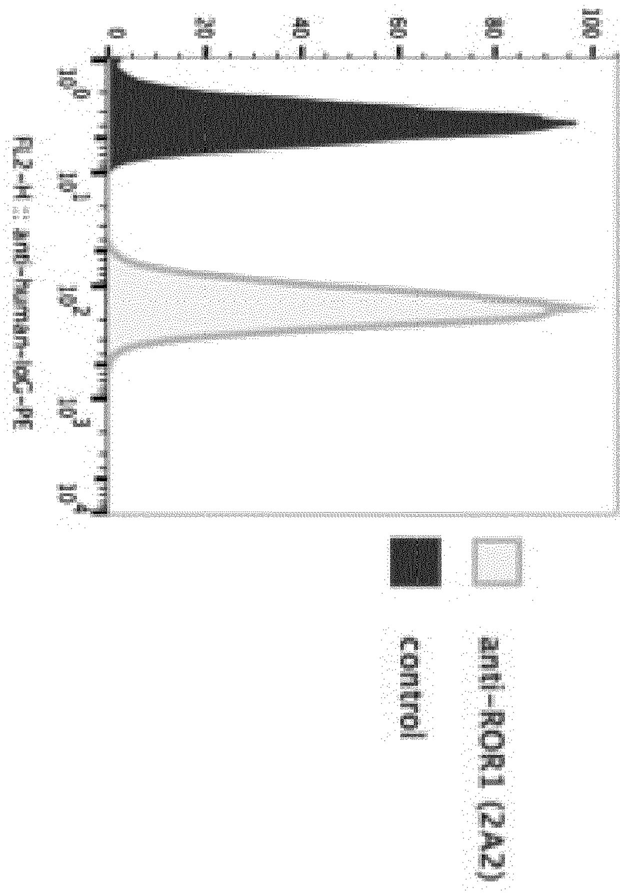

FL2-H :: anti-human-IgG-PE
0
20
40
60
80
100
10^0
10^1
10^2
10^3
10^4
control
anti-ROR1 (2A2)


Fig. 2

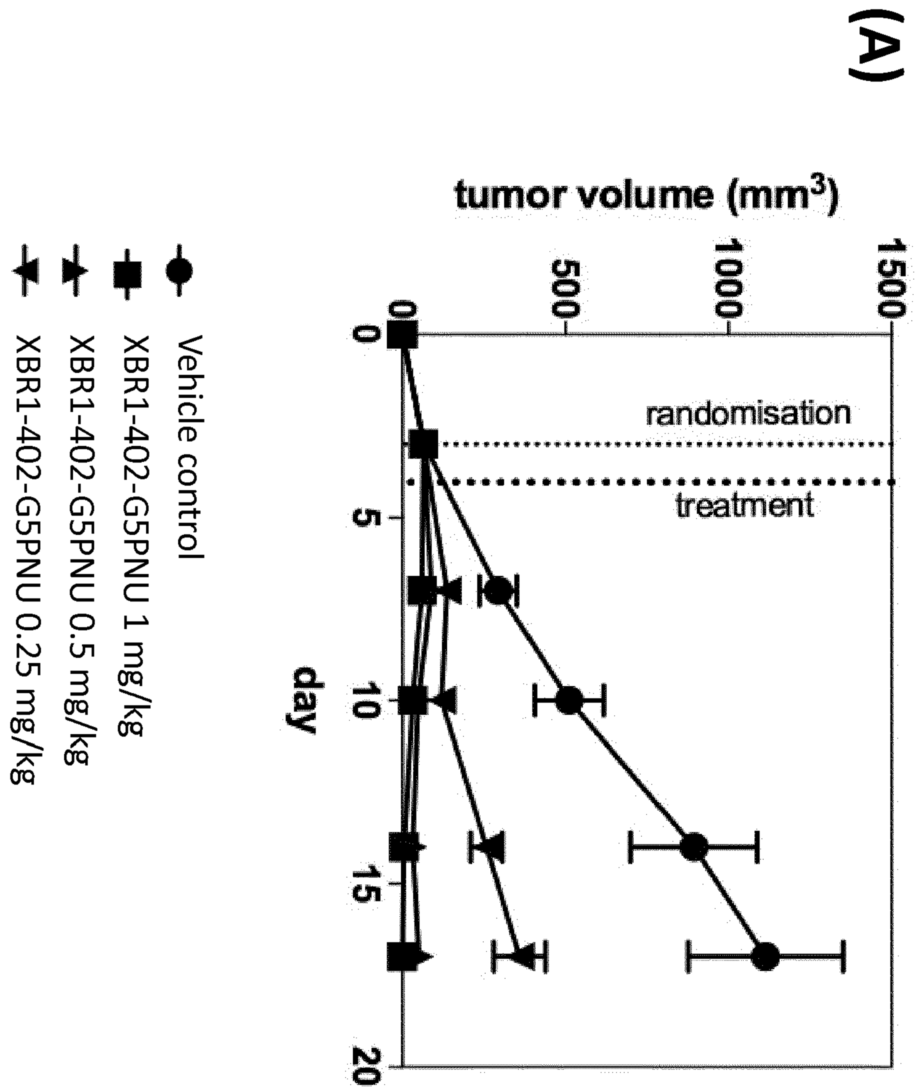
(A)
tumor volume (mm3)
1500
1000
500
0
randomisation
treatment
0
5
10
15
20
day
Vehicle control
XBR1-402-G5PNU 1 mg/kg
XBR1-402-G5PNU 0.5 mg/kg
XBR1-402-G5PNU 0.25 mg/kg

Fig. 3

(B)

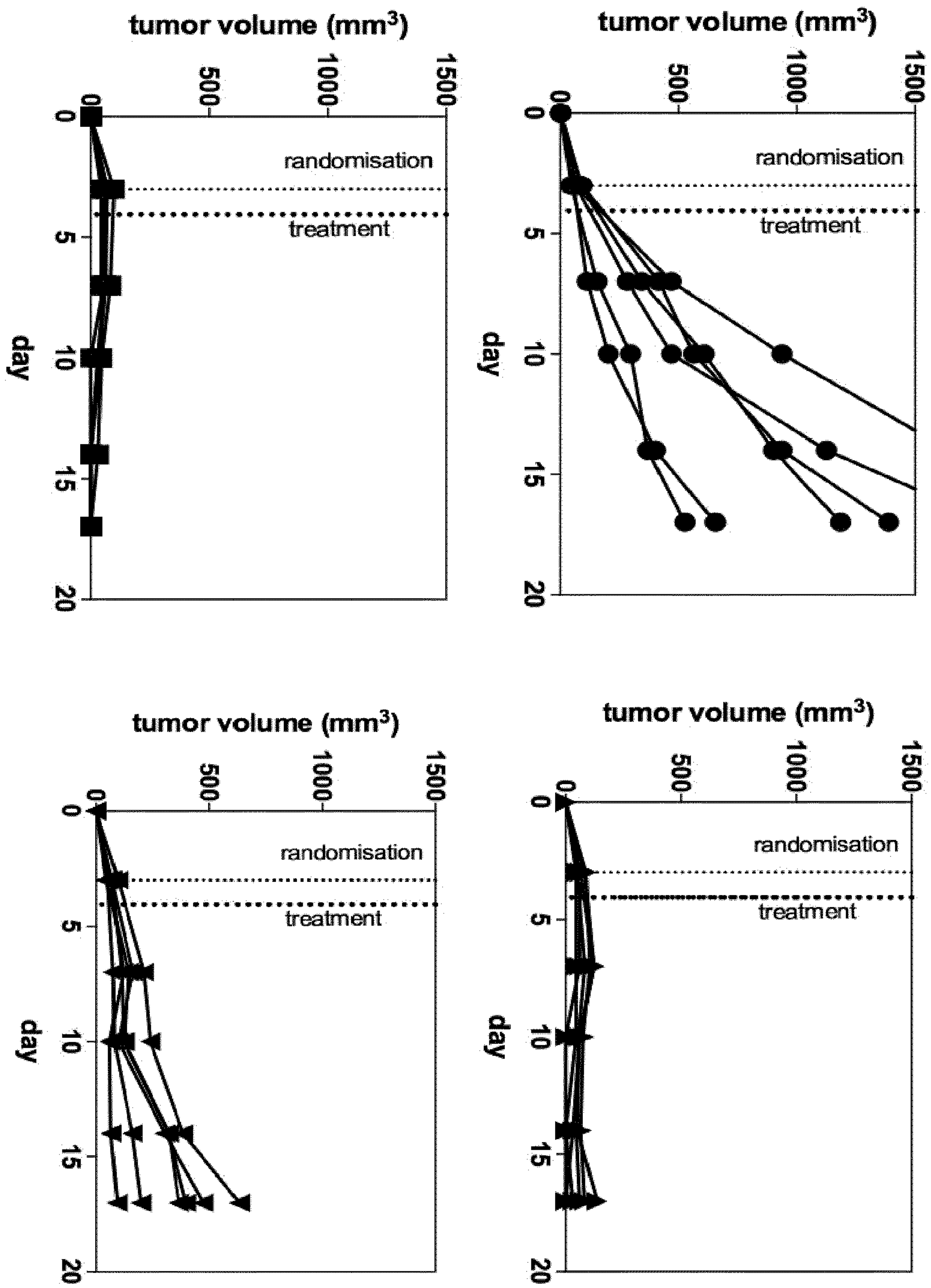
tumor volume (mm3)
0
500
1000
1500
randomisation
treatment
day
0
5
10
15
20

Fig. 3 ctd'

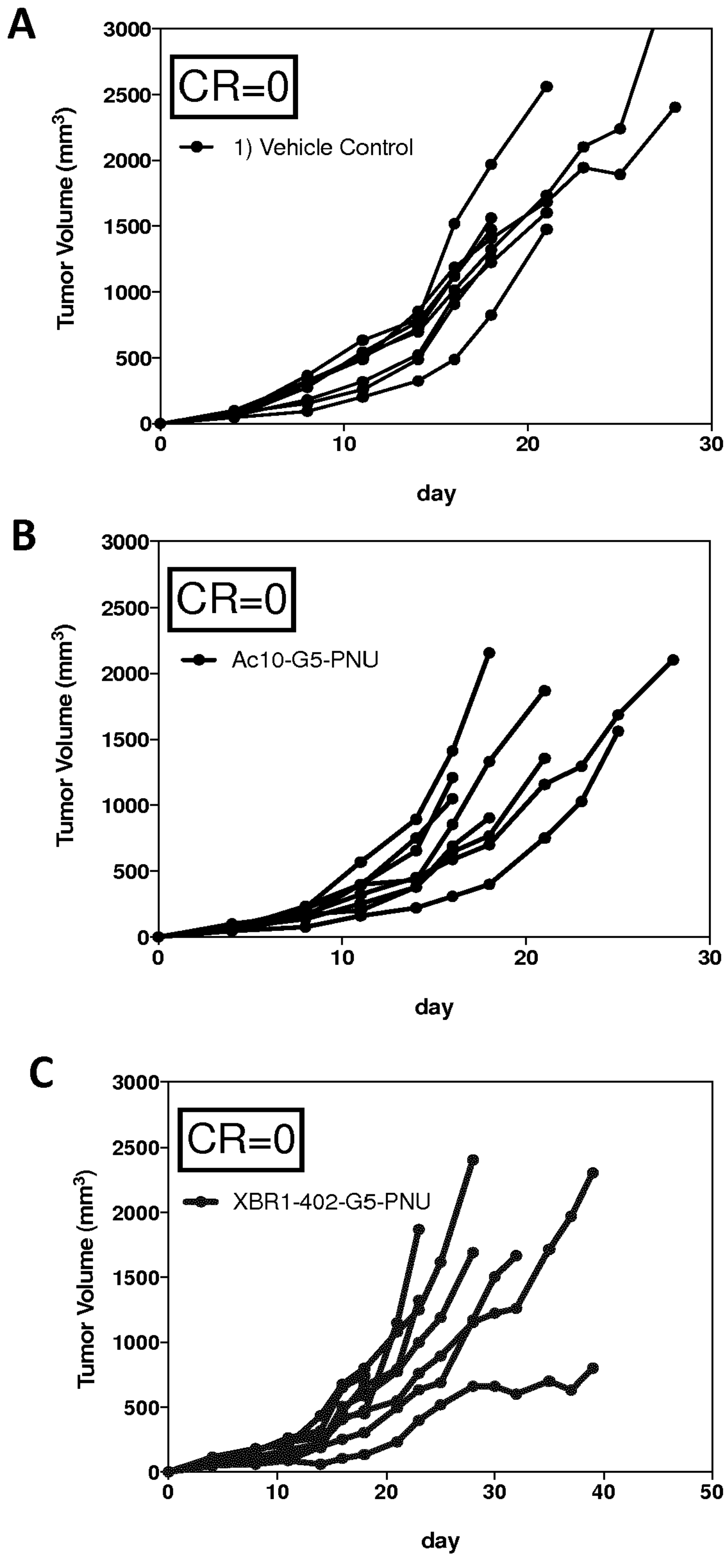
A
CR=0
1) Vehicle Control
Tumor Volume (mm3)
day
B
CR=0
Ac10-G5-PNU
Tumor Volume (mm3)
day
C
CR=0
XBR1-402-G5-PNU
Tumor Volume (mm3)
day

Fig. 4

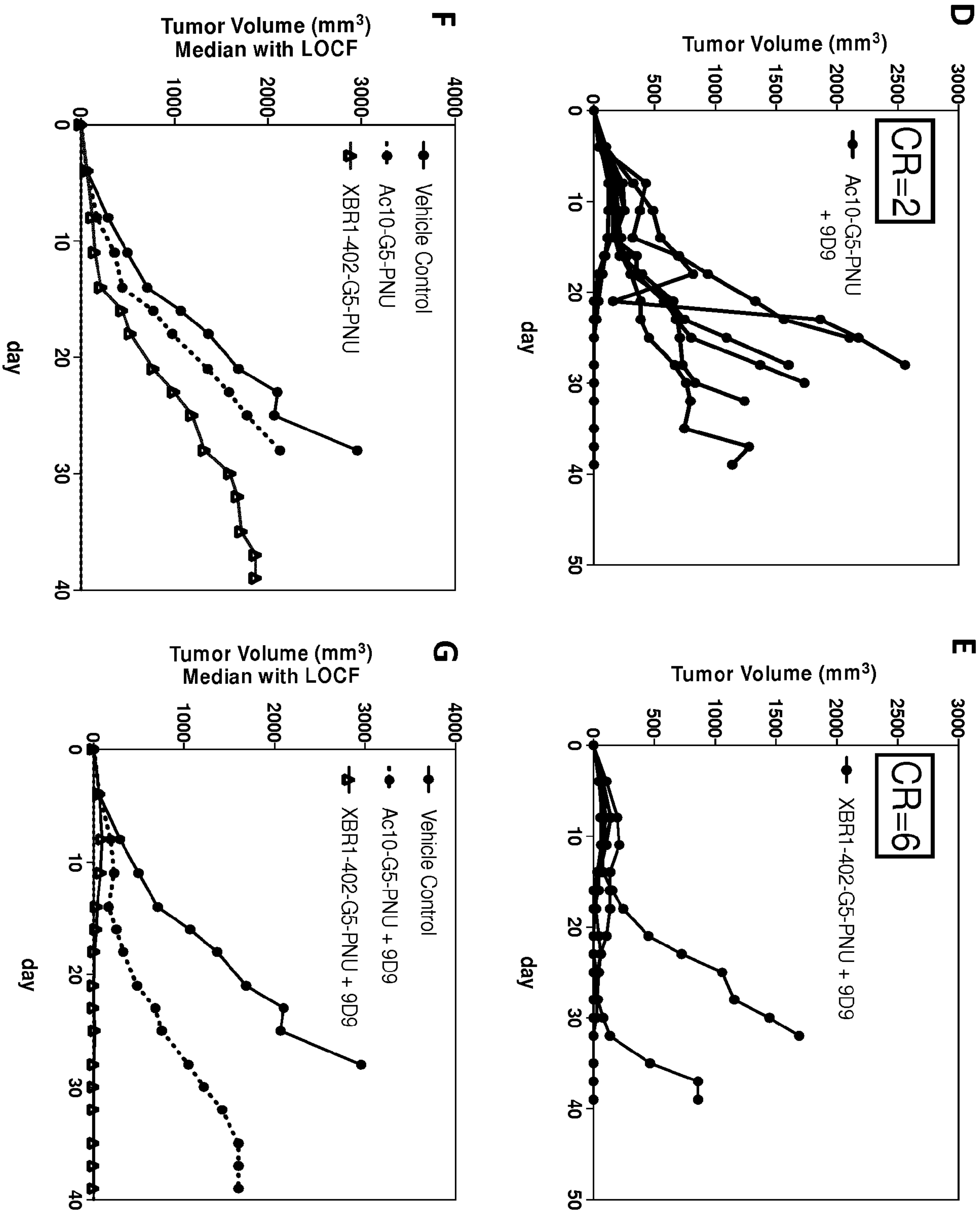
D
CR=2
Ac10-G5-PNU + 9D9
Tumor Volume (mm³)
day
E
CR=6
XBR1-402-G5-PNU + 9D9
Tumor Volume (mm³)
day
F
Vehicle Control
Ac10-G5-PNU
XBR1-402-G5-PNU
Tumor Volume (mm³) Median with LOCF
day
G
Vehicle Control
Ac10-G5-PNU + 9D9
XBR1-402-G5-PNU + 9D9
Tumor Volume (mm³) Median with LOCF
day

Fig. 4 ctd'

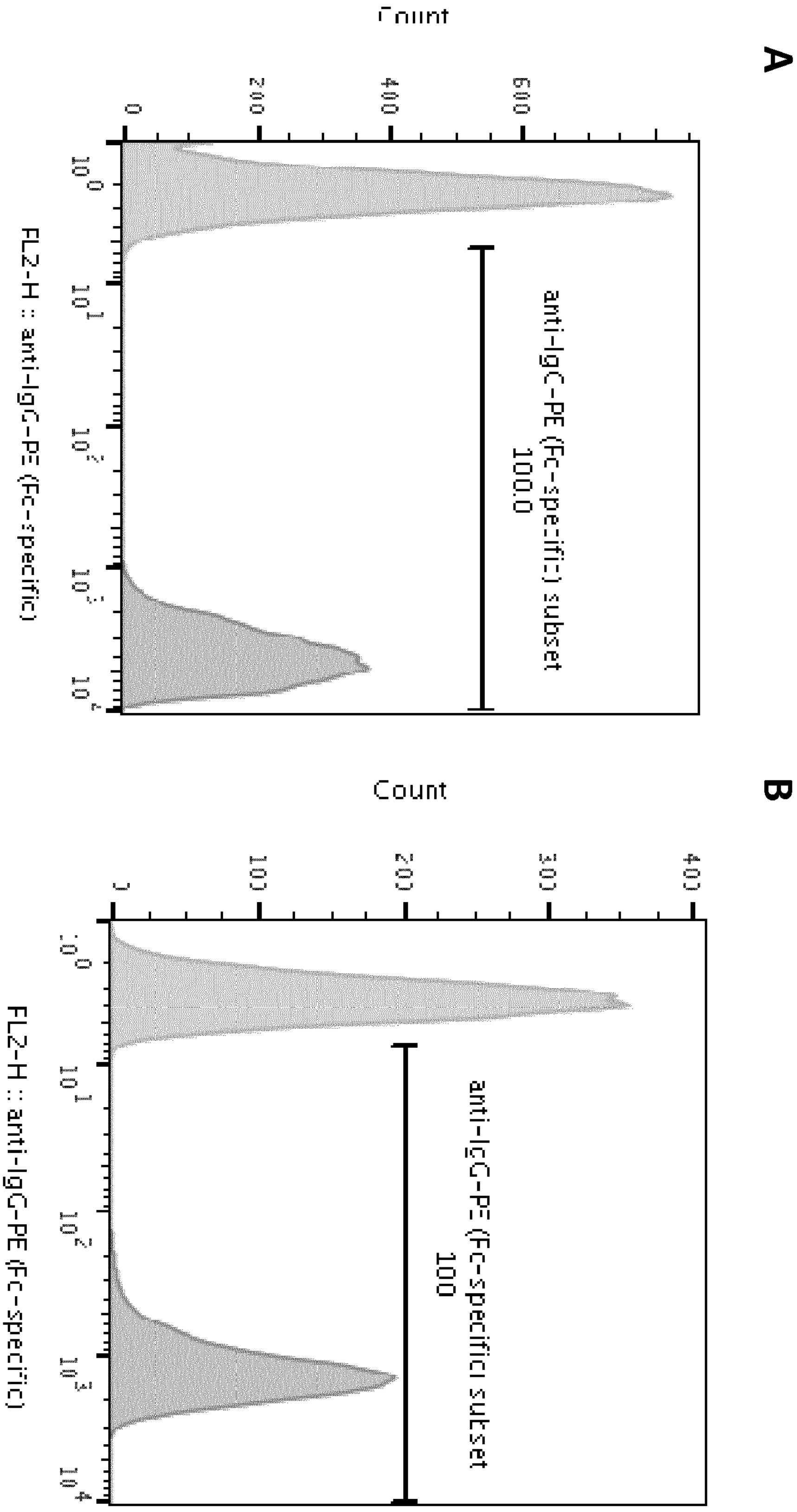
A
Count
0
200
400
600
anti-IgG-PE (Fc-specific) subset
100.0
FL2-H :: anti-IgG-PE (Fc-specific)
B
Count
0
100
200
300
400
anti-IgG-PE (Fc-specific) subset
100
FL2-H :: anti-IgG-PE (Fc-specific)

Fig. 5

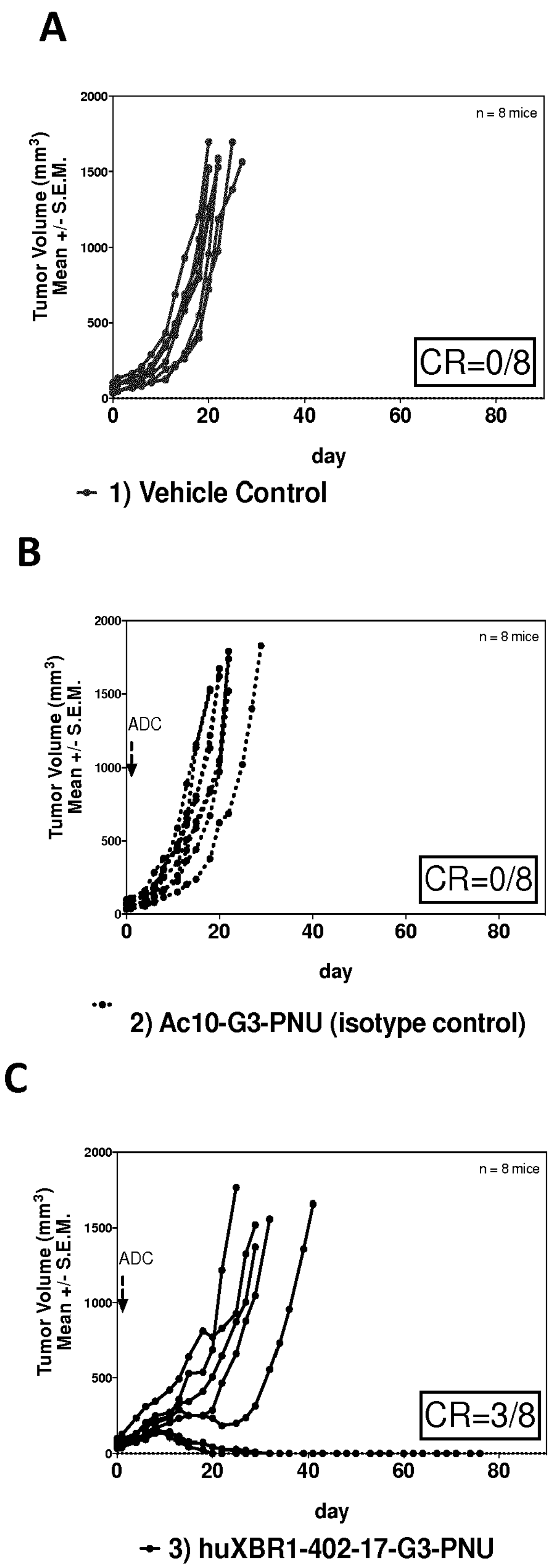
A
Tumor Volume (mm3)
Mean +/- S.E.M.
n = 8 mice
CR=0/8
day
1) Vehicle Control
B
Tumor Volume (mm3)
Mean +/- S.E.M.
n = 8 mice
ADC
CR=0/8
day
2) Ac10-G3-PNU (isotype control)
C
Tumor Volume (mm3)
Mean +/- S.E.M.
n = 8 mice
ADC
CR=3/8
day
3) huXBR1-402-17-G3-PNU

Fig. 6

D

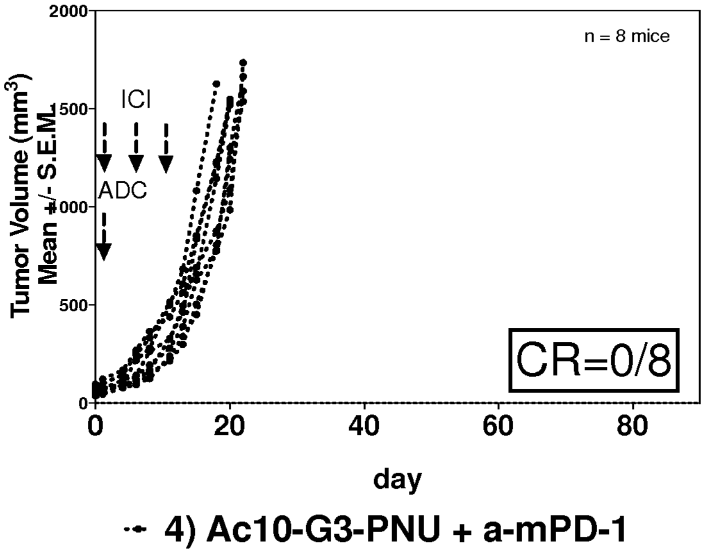
2000
1500
1000
500
0
Tumor Volume (mm3)
Mean +/- S.E.M.
n = 8 mice
ICI
ADC
CR=0/8
0
20
40
60
80
day
4) Ac10-G3-PNU + a-mPD-1

E

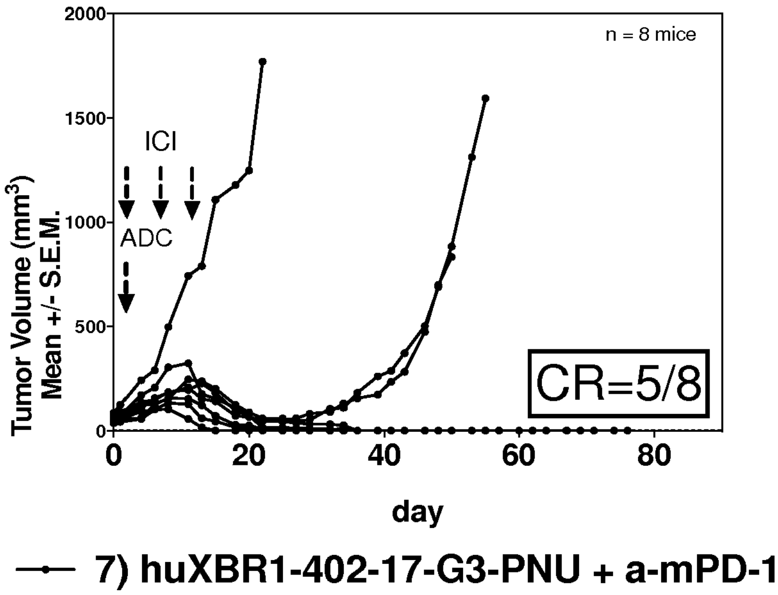
2000
1500
1000
500
0
Tumor Volume (mm3)
Mean +/- S.E.M.
n = 8 mice
ICI
ADC
CR=5/8
0
20
40
60
80
day
7) huXBR1-402-17-G3-PNU + a-mPD-1

Fig. 6 ctd'

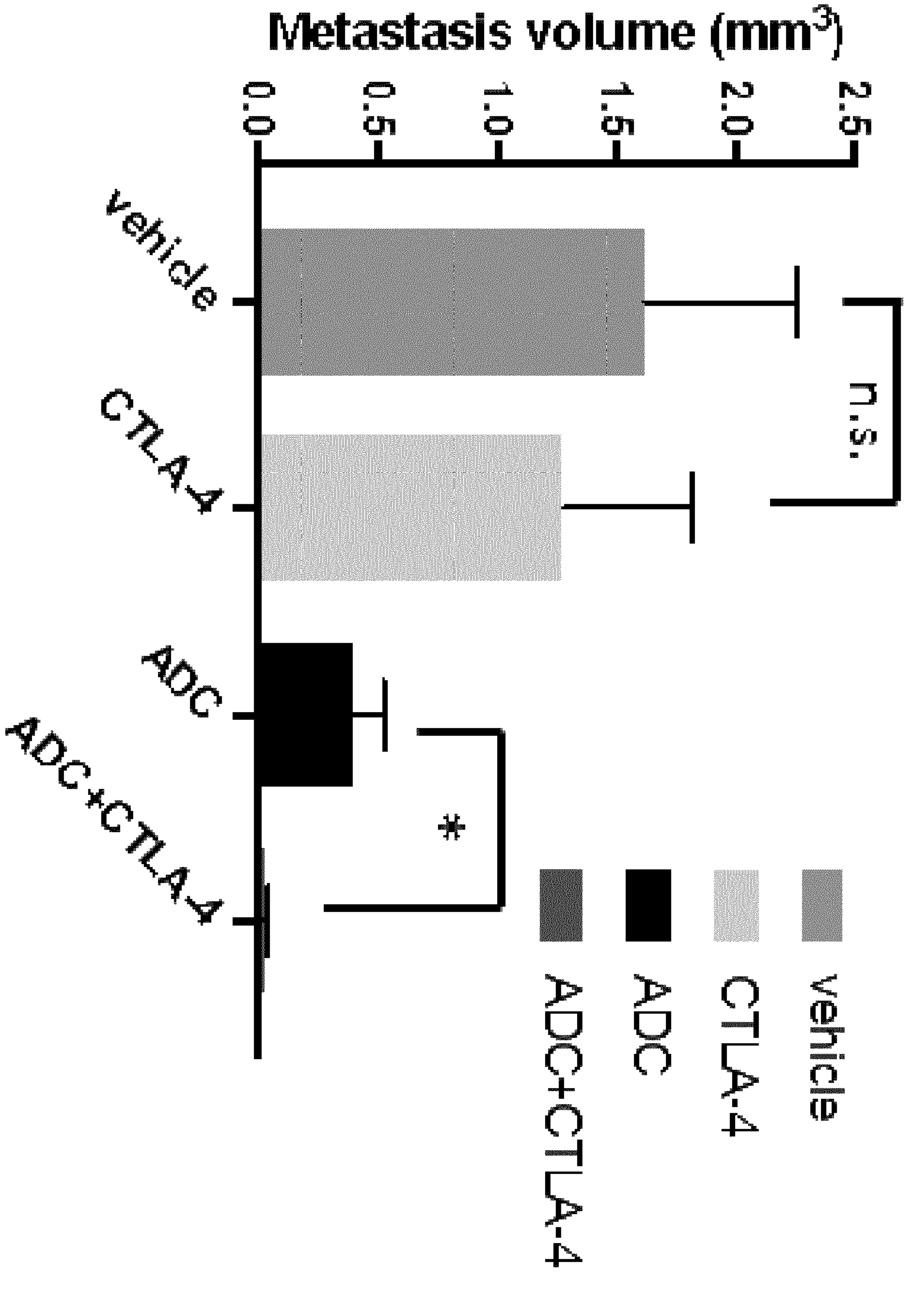
Metastasis volume (mm3)
0.0
0.5
1.0
1.5
2.0
2.5
vehicle
CTLA-4
ADC
ADC+CTLA-4
n.s.
*
vehicle
CTLA-4
ADC
ADC+CTLA-4

Fig. 7

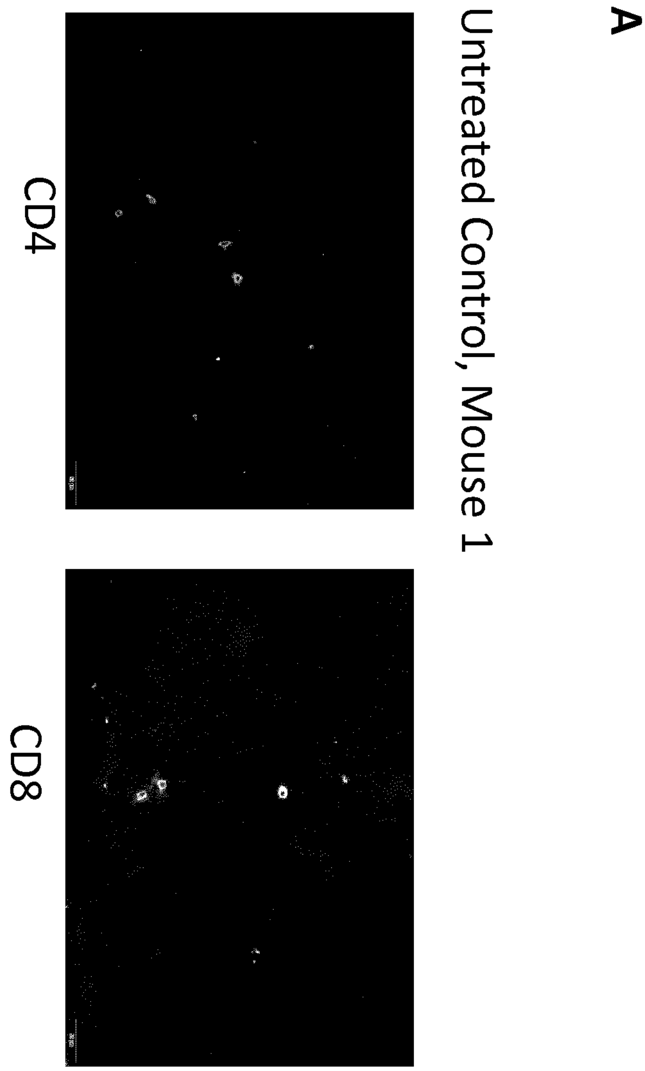


Fig. 8

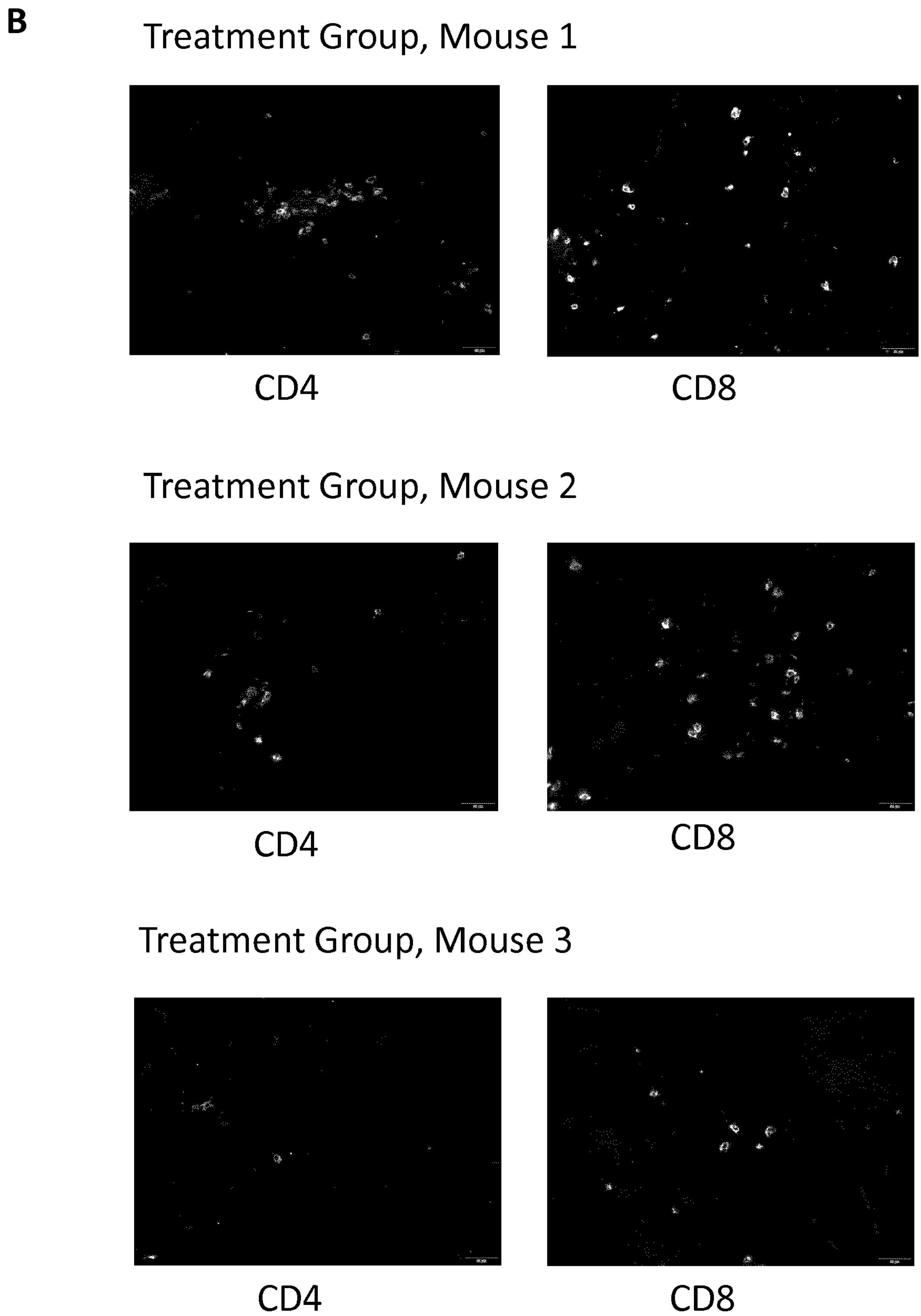
B
Treatment Group, Mouse 1
CD4
CD8
Treatment Group, Mouse 2
CD4
CD8
Treatment Group, Mouse 3
CD4
CD8

Fig. 8 ctd'

Europäisches Patentamt
European Patent Office
Office européen des brevets

# EUROPEAN SEARCH REPORT

Application Number
EP 18 19 9879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2019/016381 A1 (NBE THERAPEUTICS AG [CH]) 24 January 2019 (2019-01-24)<br>* examples 9-11 *<br>----- | 1,2,5,6,<br>8-18 | INV.<br>A61K47/68<br>A61P35/00 |
| E | WO 2019/030223 A1 (NBE THERAPEUTICS AG [CH]) 14 February 2019 (2019-02-14)<br>* examples 7-10 *<br>* figures 6-9 *<br>----- | 1,2,5,6,<br>8-18 | |
| E | WO 2018/235024 A1 (SORRENTO THERAPEUTICS INC [US]) 27 December 2018 (2018-12-27)<br>* page 15 *<br>* page 17 - page 24 *<br>* examples *<br>----- | 1,2,5,6,<br>8-18 | |
| X<br><br><br>Y | WO 2017/127664 A1 (SCRIPPS RESEARCH INST [US]; NBE-THERAPEUTICS AG [CH]) 27 July 2017 (2017-07-27)<br>* paragraph [00125] *<br>* examples 10,12 *<br>----- | 1,2,5,6,<br>8-18<br><br>3,4,7 | |
| X<br><br>Y | WO 2017/072361 A1 (NBE-THERAPEUTICS AG [CH]) 4 May 2017 (2017-05-04)<br>* figures 8,12 *<br>----- | 1,2,5,6,<br>8-18<br>3,4,7 | TECHNICAL FIELDS SEARCHED (IPC)<br>A61K<br>A61P |
| X<br><br>Y | WO 2017/127702 A1 (SCRIPPS RESEARCH INST [US]) 27 July 2017 (2017-07-27)<br>* examples 5, 9, 1-12 *<br>----- | 1,2,5,6,<br>8-18<br>3,4,7 | |
| X,D<br><br>Y | WO 2016/102679 A1 (NBE THERAPEUTICS AG [CH]) 30 June 2016 (2016-06-30)<br>* examples *<br>* figure 3A *<br>----- | 1,2,5,6,<br>8-18<br>3,4,7 | |

-/--

The present search report has been drawn up for all claims

2

| Place of search | Date of completion of the search | Examiner |
|---|---|---|
| The Hague | 17 April 2019 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 19 9879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAMUELE CAZZAMALLI ET AL: "Acetazolamide Serves as Selective Delivery Vehicle for Dipeptide-Linked Drugs to Renal Cell Carcinoma", MOLECULAR CANCER THERAPEUTICS, vol. 15, no. 12, December 2016 (2016-12), pages 2926-2935, XP055456478, ISSN: 1535-7163, DOI: 10.1158/1535-7163.MCT-16-0283 | 1,2,5,6, 8,16,18 | |
| Y | * abstract * <br> * page 2928; figure 1 * <br> * page 2932 - page 2934 * <br> * figures 4,5B,6 * <br> ----- | 3,4,7 | |
| X | NIKOLAS STEFAN ET AL: "Highly Potent, Anthracycline-based Antibody-Drug Conjugates Generated by Enzymatic, Site-specific Conjugation", MOLECULAR CANCER THERAPEUTICS, vol. 16, no. 5, 3 March 2017 (2017-03-03), pages 879-892, XP055517012, ISSN: 1535-7163, DOI: 10.1158/1535-7163.MCT-16-0688 | 1,2,5,6, 8-18 | TECHNICAL FIELDS SEARCHED (IPC) |
| Y | * abstract * <br> * page 880, right-hand column - page 881 * <br> * figure 1 * <br> * page 883, left-hand column, last paragraph - page 887, left-hand column * <br> * page 888 * <br> ----- <br> -/-- | 3,4,7 | |

The present search report has been drawn up for all claims

2

| Place of search | Date of completion of the search | Examiner |
|---|---|---|
| The Hague | 17 April 2019 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 19 9879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | AGNELLO G ET AL: "Depleting blood arginine with AEB1102 (Pegzilarginase) exerts additive anti-tumor and synergistic survival benefits when combined with immunomodulators of the PD-1 pathway", JOURNAL FOR IMMUNOTHERAPY OF CANCER, vol. 5, no. Suppl. 2, November 2017 (2017-11), page 113, XP009505820, ISSN: 2051-1426, DOI: 10.1186/S40425-017-0288-4 * abstract * ----- | 3,4,7 | |
| A | BAER SAMUEL ET AL: "Comparison of alternative nucleophiles for Sortase A-mediated bioconjugation and application in neuronal cell labelling", ORGANIC & BIOMOLECULAR CHEMISTRY, vol. 12, no. 17, 7 May 2014 (2014-05-07), pages 2675-2685, XP002725101, ISSN: 1477-0520, DOI: 10.1039/C30B42325E * the whole document * ----- | 9-15,17 | TECHNICAL FIELDS SEARCHED (IPC) |
| A,O | Ulf Grawunder: "Development of best-in-class, homogeneous Antibody Drug Conjugates (ADCs)", World ADC congress, Berlin, DE, 8-9 February 2016 , 8 February 2016 (2016-02-08), XP055341004, Retrieved from the Internet: URL:http://worldadc-europe.com/wp-content/uploads/sites/104/2016/02/Ulf-Grawunder.pdf [retrieved on 2017-02-01] * the whole document * ----- | 9-15,17 | |

The present search report has been drawn up for all claims

2

| Place of search | Date of completion of the search | Examiner |
|---|---|---|
| The Hague | 17 April 2019 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO.

EP 18 19 9879

This annex lists the patent family members relating to the patent documents cited in the above-mentioned European search report. The members are as contained in the European Patent Office EDP file on The European Patent Office is in no way liable for these particulars which are merely given for the purpose of information.

17-04-2019

| Patent document cited in search report | Publication date | Patent family member(s) | Publication date |
|---|---|---|---|
| WO 2019016381 A1 | 24-01-2019 | NONE | |
| WO 2019030223 A1 | 14-02-2019 | WO 2019030223 A1 | 14-02-2019 |
| | | WO 2019030240 A1 | 14-02-2019 |
| WO 2018235024 A1 | 27-12-2018 | US 2018360985 A1 | 20-12-2018 |
| | | WO 2018235024 A1 | 27-12-2018 |
| WO 2017127664 A1 | 27-07-2017 | AU 2017210327 A1 | 09-08-2018 |
| | | BR 112018014615 A2 | 11-12-2018 |
| | | CA 3011815 A1 | 27-07-2017 |
| | | CL 2018001971 A1 | 21-12-2018 |
| | | CN 108848669 A | 20-11-2018 |
| | | EP 3405496 A1 | 28-11-2018 |
| | | JP 2019509021 A | 04-04-2019 |
| | | KR 20180101554 A | 12-09-2018 |
| | | SG 11201806120W A | 30-08-2018 |
| | | US 2018340026 A1 | 29-11-2018 |
| | | WO 2017127664 A1 | 27-07-2017 |
| WO 2017072361 A1 | 04-05-2017 | AU 2016345681 A1 | 10-05-2018 |
| | | CA 3001941 A1 | 04-05-2017 |
| | | CN 108884160 A | 23-11-2018 |
| | | EA 201891066 A1 | 31-10-2018 |
| | | EP 3368574 A1 | 05-09-2018 |
| | | JP 2019502363 A | 31-01-2019 |
| | | KR 20180069067 A | 22-06-2018 |
| | | SG 11201803098V A | 30-05-2018 |
| | | WO 2017072361 A1 | 04-05-2017 |
| WO 2017127702 A1 | 27-07-2017 | AU 2017209313 A1 | 09-08-2018 |
| | | CA 3011817 A1 | 27-07-2017 |
| | | CN 109311982 A | 05-02-2019 |
| | | EP 3405494 A1 | 28-11-2018 |
| | | JP 2019509023 A | 04-04-2019 |
| | | KR 20180099887 A | 05-09-2018 |
| | | SG 11201806121P A | 30-08-2018 |
| | | US 2019031754 A1 | 31-01-2019 |
| | | WO 2017127702 A1 | 27-07-2017 |
| WO 2016102679 A1 | 30-06-2016 | AU 2015370918 A1 | 13-07-2017 |
| | | BR 112017013661 A2 | 13-03-2018 |
| | | CA 2971634 A1 | 30-06-2016 |
| | | CN 107750169 A | 02-03-2018 |
| | | EA 201791359 A1 | 30-11-2017 |
| | | EP 3237065 A1 | 01-11-2017 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 18 19 9879

This annex lists the patent family members relating to the patent documents cited in the above-mentioned European search report. The members are as contained in the European Patent Office EDP file on The European Patent Office is in no way liable for these particulars which are merely given for the purpose of information.

17-04-2019

| Patent document cited in search report | Publication date | Patent family member(s) | Publication date |
|---|---|---|---|
| | | JP 2018504390 A | 15-02-2018 |
| | | KR 20170106965 A | 22-09-2017 |
| | | SG 11201705041W A | 28-07-2017 |
| | | US 2017360953 A1 | 21-12-2017 |
| | | WO 2016102679 A1 | 30-06-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016102679 A
- WO 2014140317 A
- WO 2014140317 A1


### Non-patent literature cited in the description


- **WARD et al.** *Nature,* 1989, vol. 341, 544-546
- **GALON J et al.** *J Pathol,* 2014, vol. 232, 199-209
- **PAGES F et al.** *J Clin Oncol,* 2009, vol. 27, 5944-5951
- **ANGELL HK ; GALON J.** *Curr Opin Immunol,* 2013, vol. 25, 261-267
- **GALON et al.** *Immunity,* 2013, vol. 39, 11-26
- **BU X et al.** *Trends in Molecular Medicine,* 2016, vol. 22 (6), 448-451
- **SHI et al.** *Org. Biomol. Chem.,* 2009, vol. 7, 3709-3722
- **MOSELY, S. et al.** *Cancer Immunology Research,* January 2017
- **SAXENA M ; CHRISTOFORI G.** *Molecular Oncology,* 2013, vol. 7, 283-296
- **CELIK, C. et al.** *Cancer Res,* 1983, vol. 43, 3507-3510
- **GROSSO, J. et al.** *Cancer Immunity,* 2013, vol. 22 (5), 13